# EUROPEAN PATENT APPLICATION

(11) **EP 1 842 846 A1**
(43) Date of publication of application: **10.10.2007**
(21) Application number: 06007416.8
(22) Date of filing: 07.04.2006
(51) Int. Cl.: C07D 211/22, C07D 211/26, C07D 401/12, A61K 31/451, A61K 31/4523, A61P 3/00

(54) **Phenylpiperidine derivatives as melanocortin-4 receptor modulators**

(71) Applicant: Santhera Pharmaceuticals (Schweiz) AG, 4410 Liestal (CH)
(72) Inventor: Soeberdt, Michael, 79618 Rheinfelden (DE); Weyermann, Philipp, 4450 Sissach (CH); Deppe, Holger, 4053 Basel (CH); Bulat, Stephan, 79541 Lörrach (DE); von Sprecher, Andreas, 4104 Oberwil (CH); Feurer, Achim, 79424 Auggen (DE)
(74) Representative: Grünecker, Kinkeldey, Stockmair & Schwanhäusser Anwaltssozietät

(57) **Abstract**

The present invention relates to substituted phenylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

## Description

### Field of the Invention

The present invention relates to substituted phenylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1 R to MC-5R) have been identified and these are expressed in different tissues.

MC-1 R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.

The MC-2R is expressed in the adrenal gland representing the ACTH receptor. The MC-2R is not a receptor for α -MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1 R. The mechanism by which agonism of MC-1 R results in an anti-inflammatory response is likely through inhibition of the pro-inflammatory transcription activator, NF-_{K}B. NF-_{K}B is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1 R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1 R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (

H. Wessells et al., "Synthetic Melanotropic Peptide Initiates Erections in Men With Psychogenic Erectile Dysfunction: Double-Blind, Placebo Controlled Crossover Study", J. Urol., 160: 389-393, (1998)). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (M.J. Owen and C.B. Nemeroff, "Physiology and pharmacology of corticotrophin releasing factor." Pharmacol. Rev. 43: 425-473 (1991)). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Shigeyuki Chaki et al, "Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor", J. Pharm. Exp. Ther. 304(2), 818-826 (2003)).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (D.L. Marks et al. "Role of the central melanocortin system in cachexia." Cancer Res. 61: 1432-1438 (2001)).

Modulators of the melanocortin receptor are already known from the literature. WO 2004/024720 A1 describes piperazine urea derivatives which are selective agonists of the human melanocortin-4 receptor and as such they are claimed to be useful in the treatment of prevention of obesity-related disorders.

WO 2005/047253 A1 describes 4,4-disubstituted piperidine derivatives which are postulated to function as melanocortin receptor agonists.

Substituted piperidine derivatives are also described in DE 103 00973 which relates to carboxylic acids and esters having a piperidine ring or a piperazine ring as the central core of the molecule and wherein the core is further substituted in the para-position by a 5-7-membered heterocycle, a phenyl ring, a pyridine ring or a thiazole ring. Said rings are optionally substituted by an ester group. The compounds are used in the preparation of a medicament for the treatment of headaches, non-insulin dependent diabetes mellitus (NIDDM), cardiovascularic diseases, morphintolerance, diseases of the skin, inflammations, allergic rhinitis, asthma, diseases with vascular dilatation and, consequently, with reduced blood circulation in tissues, acute or preemptive treatment of menopausal hot flashes of women with an estrogen deficiency or for the treatment of pain.

In view of the unresolved deficiencies in treatment of various diseases and disorders as discussed above, it is an object of the present invention to provide novel substituted phenylpiperidine derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

### Summary of the Invention

The present invention relates to substituted phenylpiperidine derivatives of structural formula (I) 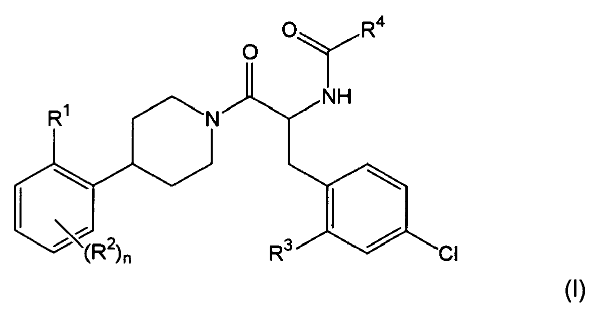 wherein R¹, R², R³, R⁴ and n are defined as described below.

The phenylpiperidine derivatives of structural formula (I) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to substituted phenylpiperidine derivatives useful as melanocortin receptor modulators, in particular, selective MC-4R agonists and MC-4R antagonists.

The compounds of the present invention are represented by structural formula (I) 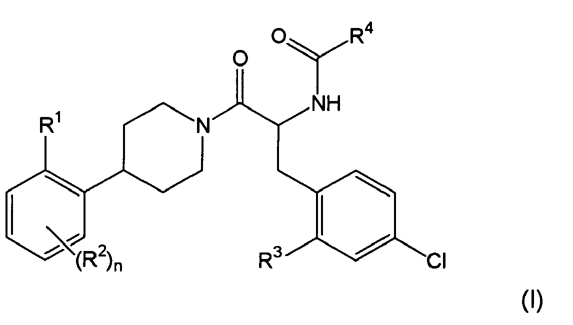 and the enantiomers, diastereomers, tautomers, solvates and pharmaceutically acceptable salts thereof,
wherein
- R¹ is: -(CH₂)ₗ-T,
-O-(CH₂)ₘ-T,
- T is: NR⁵R⁶,
morpholine,

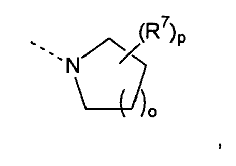

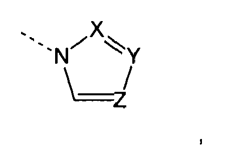

- R⁵ and R⁶: are independently
C₁₋₆-alkyl,
C₂₋₆-alkenyl,
C₂₋₆-alkinyl,
C₂₋₆-alkylene-O-C₁₋₆-alkyl,
- R⁷ is: halogen,
CN,
OH,
C₁₋₆-alkyl, optionally substituted with 1 to 3 substituents selected from halogen,
CN and OH,
O-C₁₋₆-alkyl, optionally substituted with 1 to 3 substituents selected from
halogen, CN and OH,
- X is: CH, N,
- Y is: CH, N,
- Z is: CH, N,
- R² is: F,
Cl,
methyl,
CF₃,
- R³ is: H
Cl,
methyl,
- R⁴ is: NR⁸R⁹, 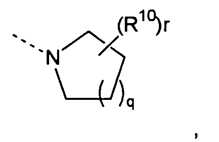 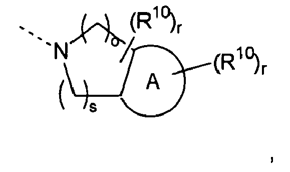
morpholine optionally substituted with 1 to 3, same or different substituents R¹¹,
- R⁸ and R⁹: are independently
C₁₋₆-alkyl,
C₂₋₆-alkenyl,
C₂₋₆-alkinyl,
C₂₋₆-alkylene-O-C₁₋₆-alkyl,
- R¹⁰ is: halogen,
CN,
OH,
C₁₋₆-alkyl optionally substituted with 1 to 3 substituents selected from halogen,
CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl),
-N(C₁₋₆-alkyl)₂,
- R¹¹ is: C₁₋₆-alkyl,
C₁₋₆ alkylene-O-C₁₋₆-alkyl,
C₁₋₆-alkylene-OH,
C₁₋₆-alkylene-NH₂,
C₁₋₆-alkylene-NH-C₁₋₆-alkyl,
C₁₋₆-alkylene-N(C₁₋₆-alkyl)₂,
- A is: a 3-7-membered, saturated, unsaturated or aromatic ring containing 0-2
nitrogen atoms,
- I is: 1,2,3,4,
- m is: 2, 3, 4,
- n is: 0, 1, 2, 3, 4,
- o is: 0, 1, 2,
- p is: 0, 1, 2, 3, 4,
- q is: 0, 1, 2, 3,
- r is: 0, 1, 2, 3, 4 and
- s is: 1,2.

Preferably, the compounds according to formula (I) adopt the structural conformation of the following stereoisomer formula (I'):

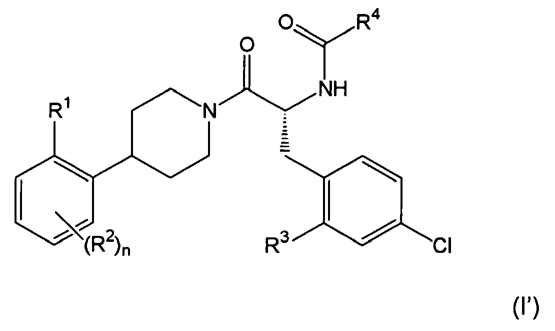

In a preferred embodiment, R² represents Cl of F. It is further preferred that R³ represents Cl. Preferably, the phenyl ring directly connected with the piperidine ring is monosubstituted by a chlorine or fluorine atom in the meta or para-position.

In a further preferred embodiment at least one of R⁵ and R⁶ is selected from C₂₋₆-alkenyl, C₂₋₆-alkinyl and C₂₋₆-alkylene-O-C₁₋₆-alkyl.

The variant I is preferably selected from 2 or 3.

The variant m is preferably selected from 2 or 3.

As regards compounds of formula (I), T is preferably selected from the group consisting of the following radicals:

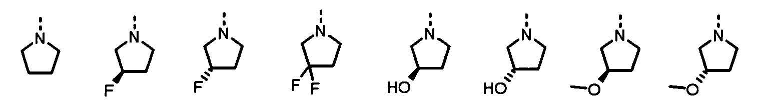

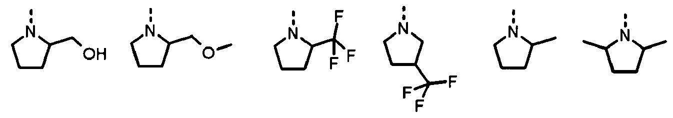

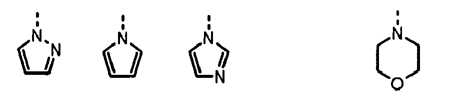

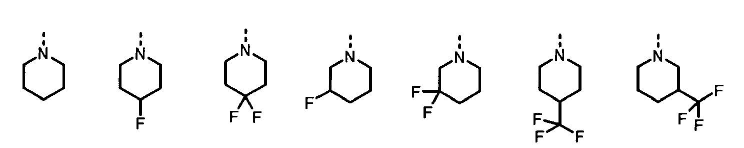

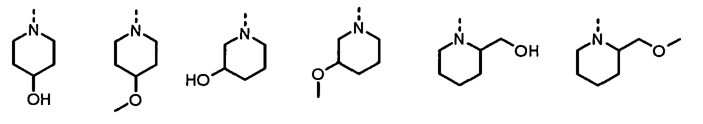

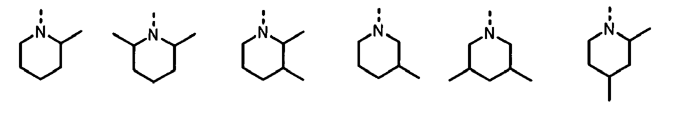

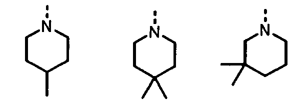

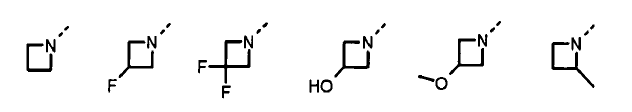

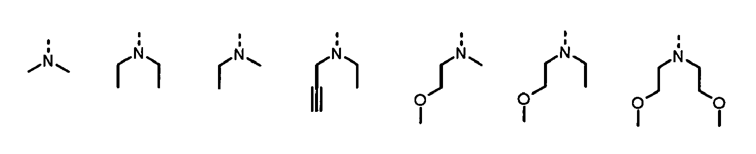

In a further preferred embodiment, R⁴ is preferably selected from the group consisting of

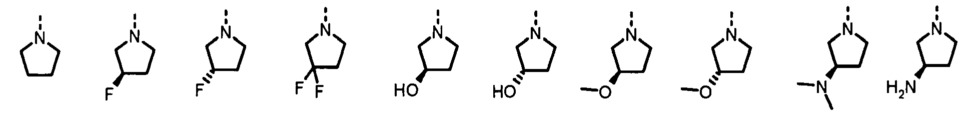

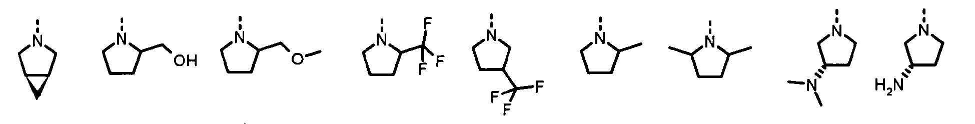

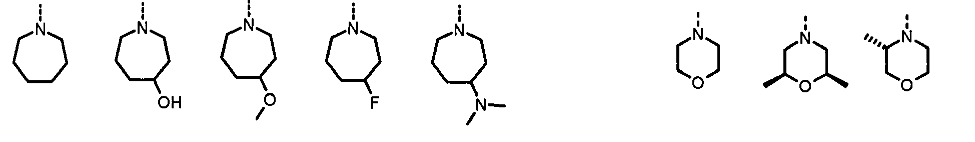

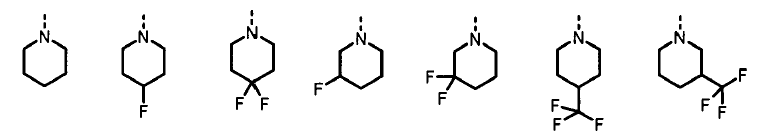

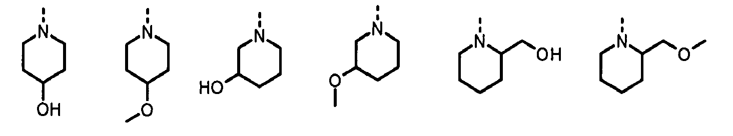

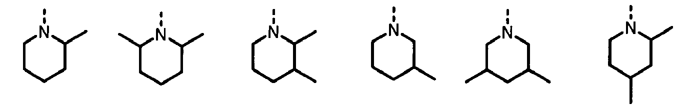

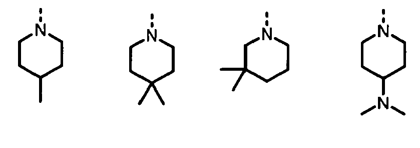

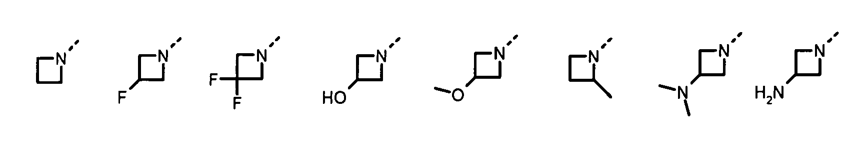

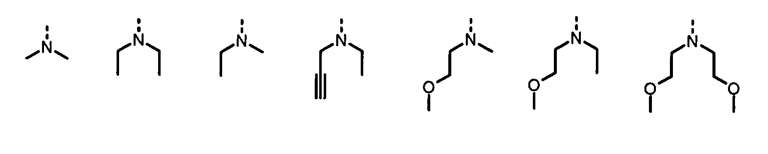

Compounds of the formula (I) in which some or all of the above-mentioned groups have the preferred or more preferred meanings are also an object of the present invention.

In the above and the following, the employed terms have the meaning as described below:

Alkyl is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.

Alkinyl is a straight chain or branched alkyl having 2 to 6 carbon atoms and which contains at least one carbon-carbon triple bond, such as ethinyl, 1-propinyl, 1-butinyl, 2-butinyl, pentinyl or hexinyl.

A 3-7-membered, saturated, unsaturated or aromatic ring containing 0-2 nitrogen atoms encompasses a 3-7-membered saturated carbocycle such as cyclopropyl, cyclobutyl, cyclopentyl, cyclohexyl or cycloheptyl. Said term further encompasses 3-7-membered unsaturated carbocycles such as cyclobutenyl, cyclopentenyl, cyclohexenyl, cycloheptenyl, cyclohexa-1,4-diene or cycloheptadienes, or aromatic rings such as benzene. Nitrogen-containing, 3-7-membered, saturated, unsaturated or aromatic heterocycles are further encompassed by the above term. Examples thereof include azetidine, pyrrolidine, piperidine, azepane, piperazine, pyridine, pyrimidine, pyrazine, pyrrole, imidazole, and pyrazole.

The compounds of structural formula (I) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

The compounds of structural formula (I) are particularly useful as antagonists of MC-4R. Thus, they are preferably used for the preparation of a medicament for the treatment and/or prevention of cancer cachexia, muscle wasting, anorexia, anxiety and depression.

### Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

### Utility

The compounds of formula (I) are melanocortin receptor modulators and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance) and male and female sexual dysfunction (including impotence, loss of libido and erectile dysfunction).

The compounds of formulas (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formula (I) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be at solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formula (I), the terms "A moiety", "B moiety" and "C moiety" are used below. This moiety concept is illustrated below:

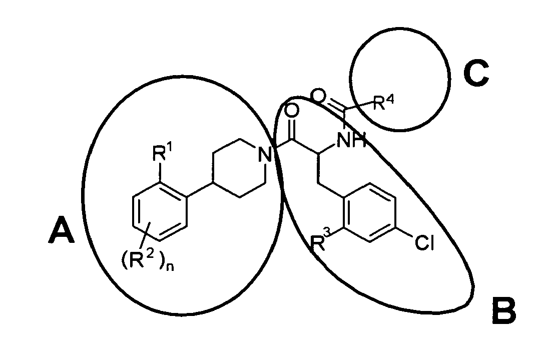

The preparation of the compounds of the present invention may be carried out via sequential or convergent synthetic routes. The skilled artisan will recognize that, in general, the A and B moieties of a compound of formula (I) are connected via amide bonds. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The B and C moieties of a compound of formula (I) are linked together via a urea function. The skilled artist can, therefore, readily envision numerous routes and methods of connecting the two moieties using different well known methods.

The compounds of formula (I), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following schemes and examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:
- AcOH: acetic acid
- Boc: tert-butoxycarbonyl
- Boc₂O: di-tert-butyl dicarbonate
- Bz₂O₂: dibenzoylperoxide
- DCM: dichloromethane
- DEAD: diethyl azodicarboxylate
- DIAD: diisopropyl azodicarboxylate
- DIEA: ethyl-diisopropylamine
- DMAP: 4-dimethylaminopyridine
- DMF: N,N-dimethylformamide
- DMS: dimethyl sulfide
- dppf: 1,1'-bis(diphenylphosphino)-ferrocen
- EDCI: 1-(3-dimethylaminopropyl)-3-ethylcarbodiimide hydrochloride
- EtOAc: ethyl acetate
- Et₂O: diethyl ether
- EtOH: ethanol
- HOBt: 1-hydroxybenzotriazole
- h: hour(s)
- MeCN: acetonitrile
- MeOH: methanol
- NBS: N-bromosuccinimide
- NMM: N-methylmorpholine
- PPh₃: triphenylphosphine
- TFA: trifluoroacetic acid
- THF: tetrahydrofurane

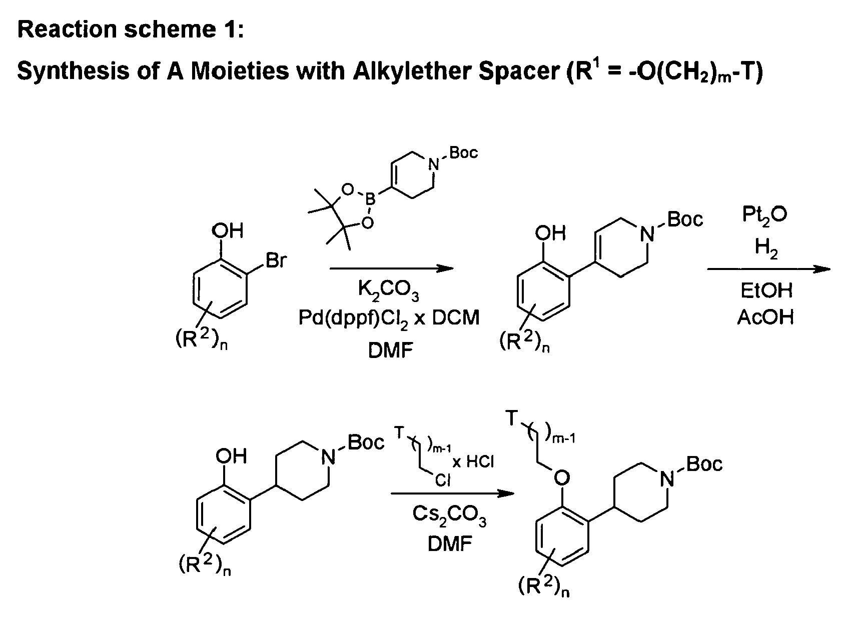

As shown in Reaction Scheme 1, optionally substituted 2-bromo-phenol and 4-(4,4,5,5-tetramethyl-[1,3,2]dioxaborolan-2-yl)-3,6-dihydro-2H-pyridine-1-carboxylic acid tert-butyl ester *(*Tetrahedron Lett. 2000, 41, 3705-3708) are reacted in a Suzuki coupling in the presence of a base such as K₂CO₃ and a catalyst such as dichloro(1,1'-bis (diphenylphosphino)-ferrocene)palladium(II) DCM adduct, in an organic solvent such as DMF or toluene, at a suitable temperature. The resulting tetrahydropyridine can be hydrogenated in the presence of a catalyst, such as PtO₂ or Pd/C, to yield the protected piperidine. The piperidine is further reacted with an alkylchloride or alkylbromide bearing the capping group T in the presence of a base such as Cs₂CO₃ or NaH in an appropriate solvent such as DMF to give the Boc-protected A moiety.

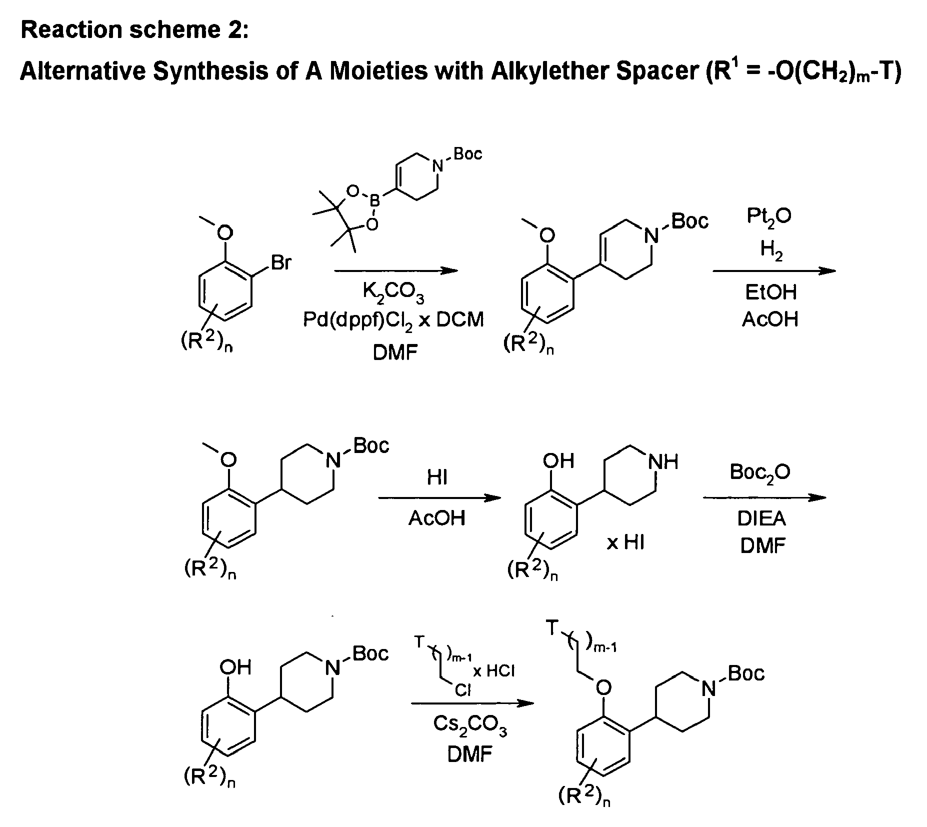

The synthesis of A moieties bearing an alkylether spacer (R¹ = -O(CH₂)ₘ-T) can alternatively be performed starting from optionally substituted 2-bromoanisole (see Reaction scheme 2). A Suzuki coupling with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature leads to the corresponding tetrahydropyridine. The resulting tetrahydropyridine can be hydrogenated in the presence of a catalyst, such as PtO₂ or Pd/C, to yield the protected piperidine. The methylether can be cleaved with a reagent such as aqueous hydroiodic acid in acetic acid or trimethylsilyl iodide in chloroform, at a suitable temperature to get access to the corresponding phenol as hydroiodide. The Boc-protecting group, which is lost during this process, can subsequently be reintroduced by using a reagent such as Boc₂O in the presence of a base such as DIEA in an appropriate solvent such as DMF or DCM. The Boc-protected piperidine is further reacted with an alkylchloride or alkylbromide bearing the capping group T in the presence of a base such as Cs₂CO₃ or NaH in an appropriate solvent such as DMF to give the Boc-protected A moiety.

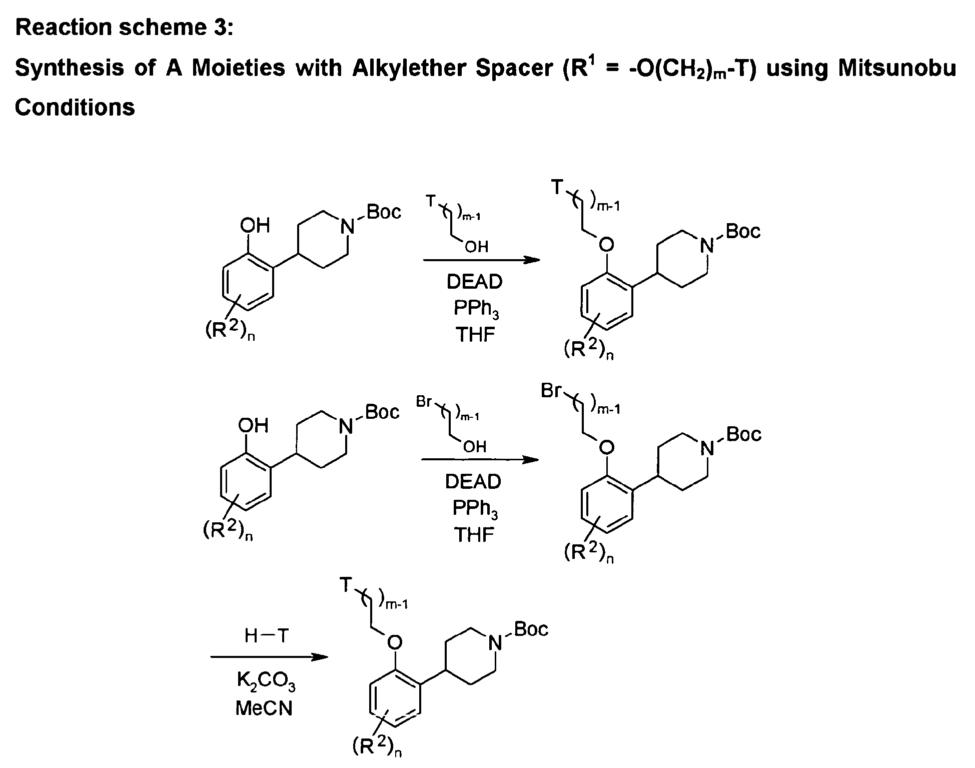

As shown in Reaction scheme 3, the intermediate product from Reaction schemes 1 and 2, optionally substituted 1-Boc-4-(2-hydroxy-phenyl)-piperidine, can also be alkylated with an ω-T-capped alkylalcohol in the presence of a reagent such as DEAD or DIAD and a phosphine such as PPh₃ in a suitable solvent such as THF to give the Boc-protected A moieties.
Similarly, the same intermediate can be reacted with an ω-bromo alkylalcohol, using the reaction conditions described above, to give access to the corresponding phenolether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

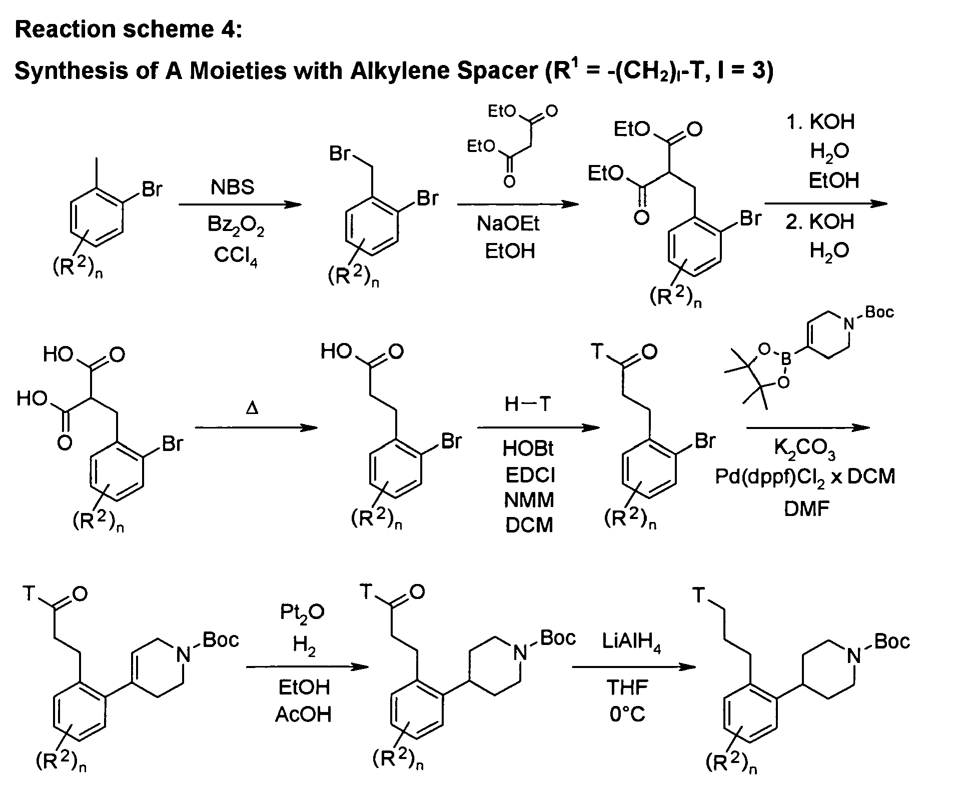

The first route for the synthesis of A moieties bearing an alkylene spacer (R¹ = -(CH₂)ₗ-T) is depicted in Reaction scheme 4. Optionally substituted 2-bromotoluene is brominated with NBS in the presence of a radical starter such as Bz₂O₂ in an appropriate solvent such as CCl₄ at a suitable temperature to yield the corresponding benzylbromide. The benzylbromide is reacted with diethyl malonate in the presence of a base such as sodium ethoxide in a suitable solvent such as ethanol. Subsequent saponification with a base such as KOH in an appropriate solvent such as water-ethanol mixture followed by a second saponification step with a suitable base such as KOH in a solvent such as water leads to the alkylated malonic acid which is decarboxylated at an appropriate temperature. The product of this reaction, optionally substituted 3-(2-bromophenyl)propionic acid, is activated with a reagent such as EDCI in the presence of a catalyst such as DMAP and a base such as NMM in DCM, and reacted with the capping group T to form the corresponding amide. Optionally substituted 3-(2-bromophenyl)propionic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

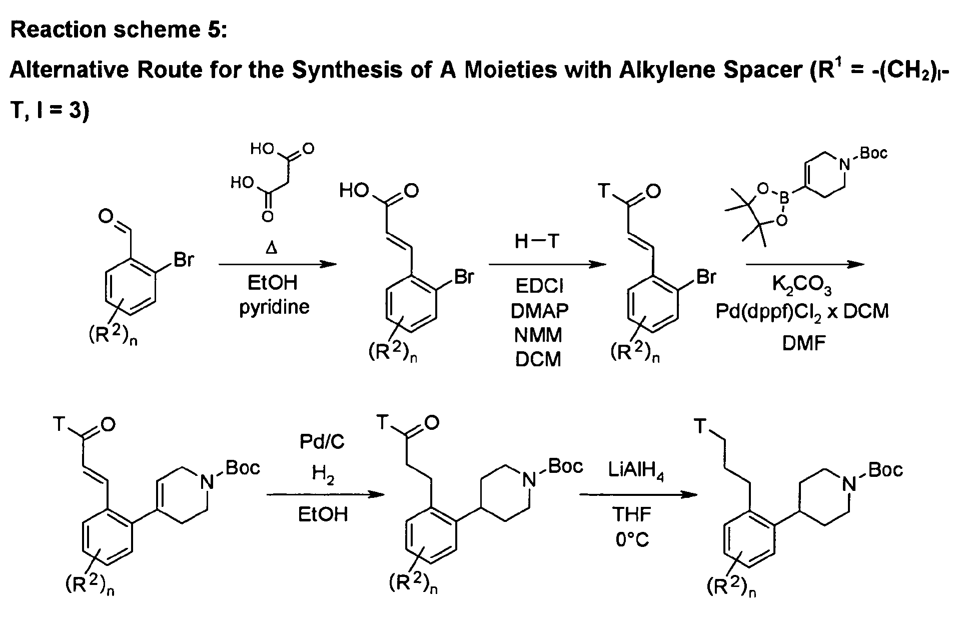

An alternative approach for the synthesis of of A moieties bearing an alkylene spacer (R¹ = -(CH₂)₁-T) starts with optionally substituted 2-bromobenzaldehyde (see Reaction scheme 5). Reaction with malonic acid in an appropriate solvent such as ethanol, in the presence of a base such as pyridine, at a suitable temperature, leads to the corresponding 2'-bromo-cinnamic acid. Said acid is activated with a reagent such as EDCI in the presence of a catalyst such as DMAP and a base such as NMM in DCM, and reacted with the capping group T to form the corresponding amide. Optionally substituted 2'-bromo-cinnamic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine and the cinnamic acid amide double bond can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

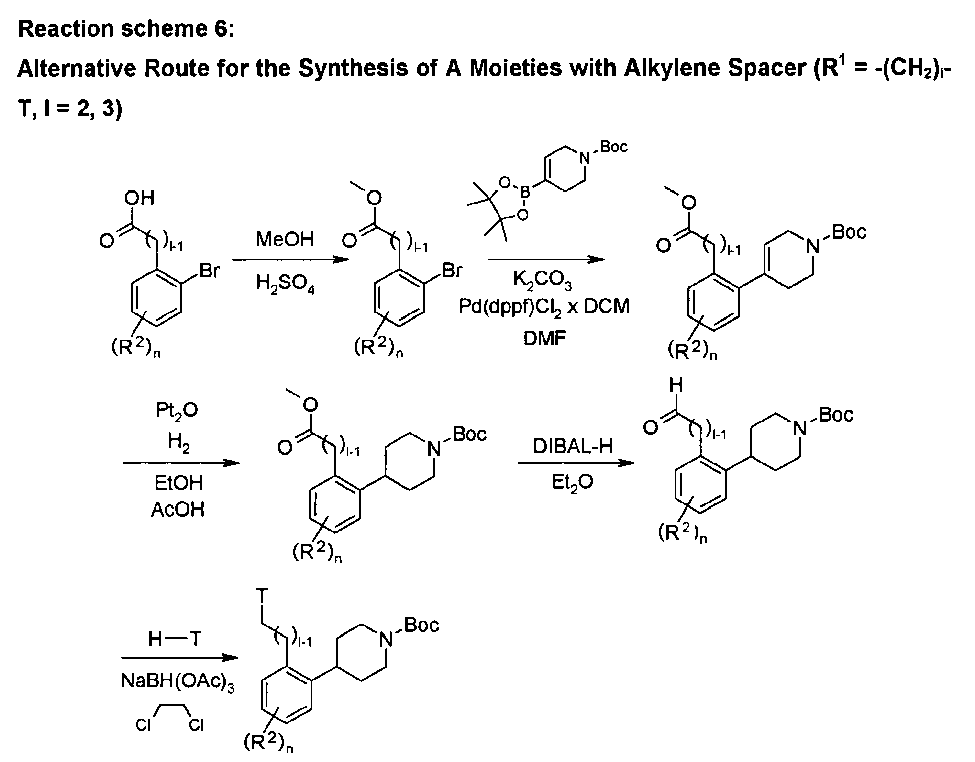

As shown in Reaction scheme 6 optionally substituted 3-(2-bromophenyl)propionic acid or 2-(2-bromophenyl)acetic acid is transformed to the corresponding methyl ester using a catalyst such as sulfuric acid. The ester can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The ester function can then be reduced to the corresponding aldehyde with DIBAL-H in an appropriate solvent such as Et₂O or THF at a suitable temperature. The aldehyde can also be obtained using a two step synthesis. Reduction of the ester with a reagent such as LiAIH₄ or borane-tetrahydrofurane complex in an inert solvent such as Et₂O or THF at an appropriate temperature leads to the corresponding alcohol which can subsequently being oxidized to the aldehyde using Swern conditions. Reductive amination of the aldehyde with an amine T-H in the presence of a reducing agent such as sodium triacetoxyborohydride in an appropriate solvent such as 1,2-dichloroethane leads to the Boc-protected A moiety.

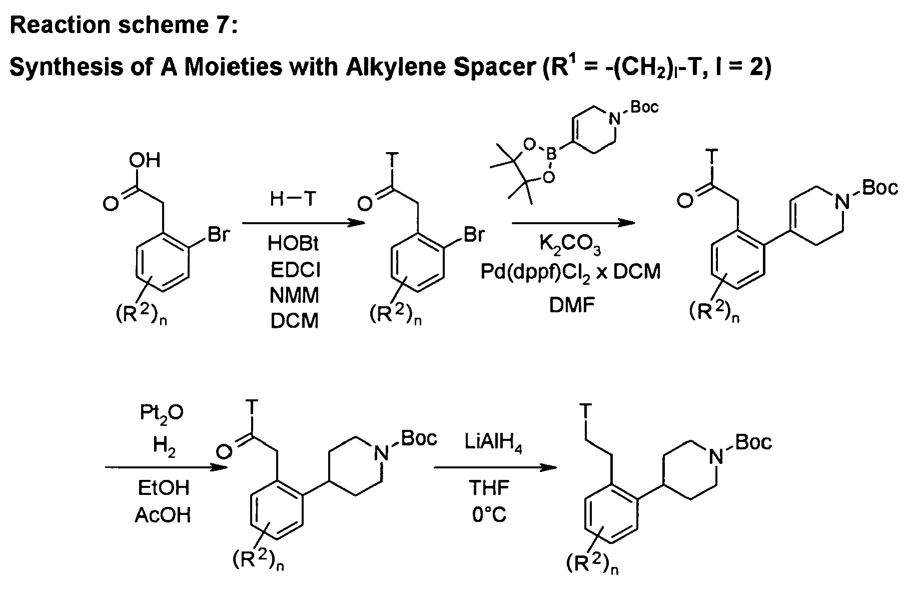

Synthesis of A Moieties with Alkylene Spacer (R¹ = -(CH₂)₁-T, I = 2) can also be performed as described in Reaction scheme 7. Optionally substituted 2'-bromophenylacetic acid is activated with a reagent such as EDCI in the presence of a catalyst such as DMAP and a base such as NMM in DCM, and reacted with the capping group T to form the corresponding amide. Optionally substituted 2'-bromo-phenylacetic amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

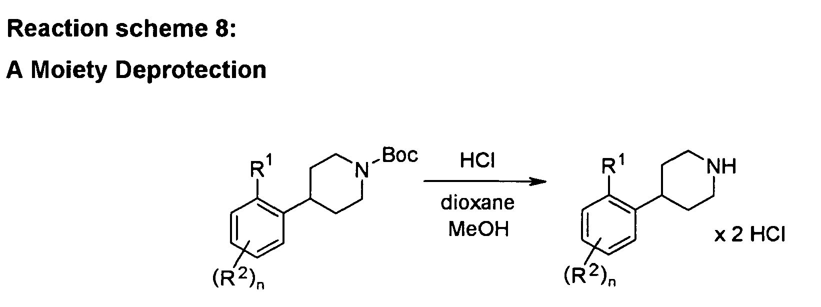

Generally, the starting material of Boc-protected phenylpiperidine (A moiety) can be deprotected in the presence of TFA/CH₂Cl₂, HCl/EtOAc, HCl/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

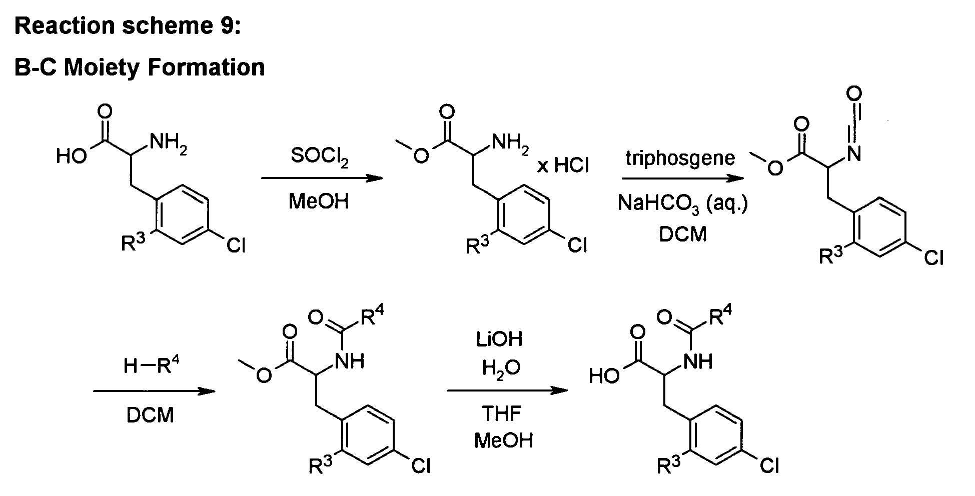

The B-C moieties can be synthesized as shown in Reaction scheme 9. Optionally substituted p-chlorophenylalanine can be converted to the corresponding methylester hydrochloride using an activating reagent such as thionyl chloride or oxalyl chloride in methanol. Amino acid methylester hydrochloride can be reacted with a reagent such as triphosgene in the presence of a base such as NaHCO₃ (aq.) in a suitable solvent such as DCM to yield the isocyanide which can subsequently be reacted with an amine R⁴-H in a suitable solvent such as DCM. The ester function can be hydrolyzed with a base such as LiOH in a suitable solvent or solvent mixture such as water/THF/methanol to give access to the B-C-moiety.

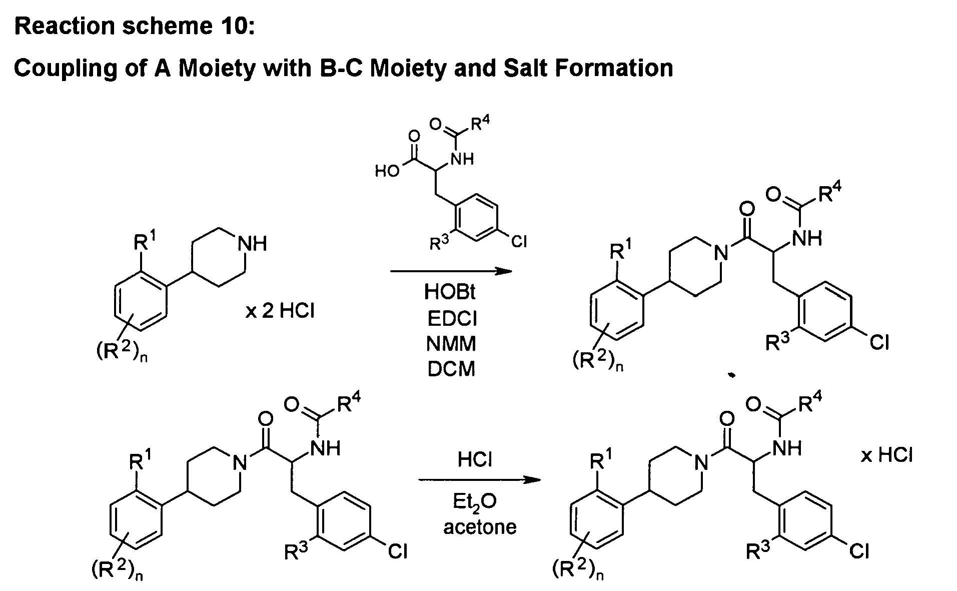

As shown in Reaction scheme 10, A moieties can be coupled with B-C moieties in the presence of EDCI/HOBt, a base such as N-methylmorpholine (NMM) and a solvent such as dichloromethane (DCM). A suitable solvent, such as DCM, DMF, THF or a mixture of the above solvents, can be used for the coupling procedure. Suitable base include triethylamine (TEA), diisopropylethylamine (DIEA), N-methylmorpholine (NMM), collidine or 2,6-lutidine. A base may not be needed when EDCI/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

The product can be transferred to a pharmaceutically acceptable salt such as a hydrochloride, using HCl in a solvent or solvent mixture such as diethyl ether/acetone.

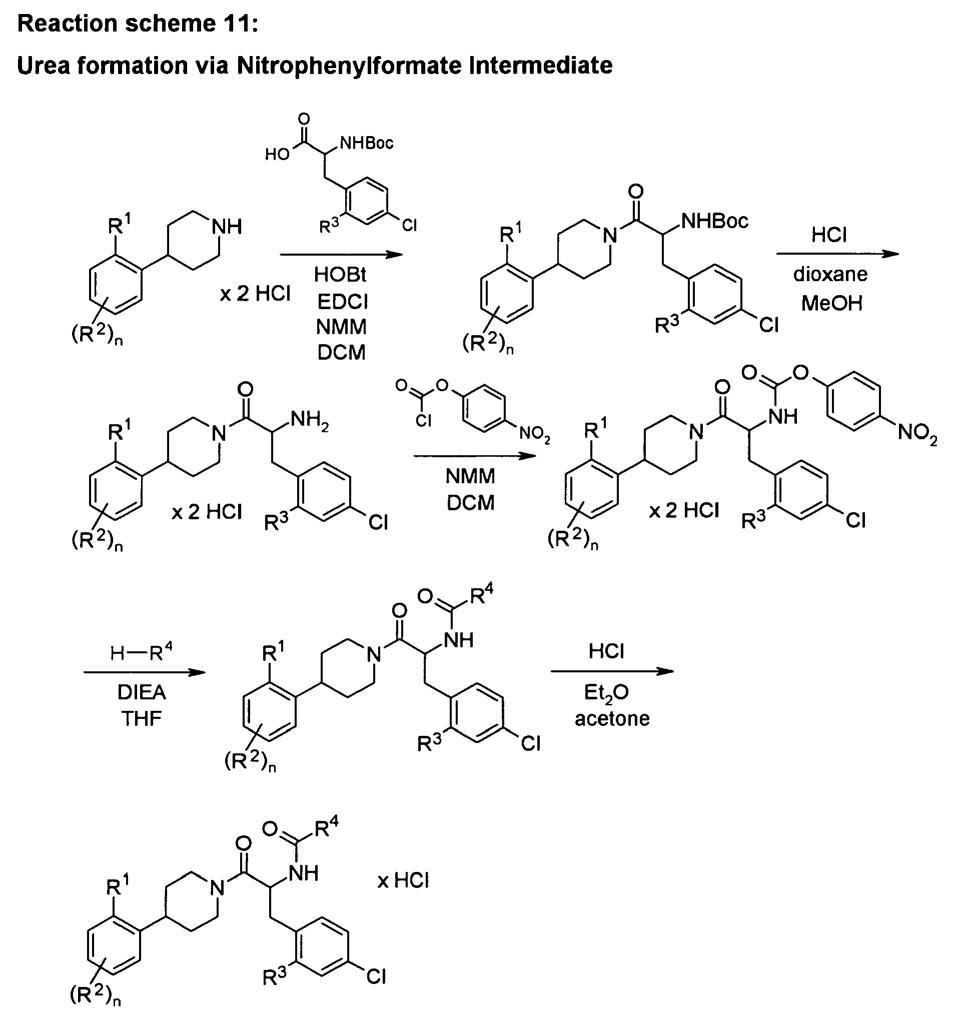

The three moieties can also be combined stepwise, as shown in reaction scheme 11. An appropriate A moiety is coupled to a Boc-protected B moiety in the presence of EDCI/HOBt, a base such as N-methylmorpholine (NMM) and a solvent such as dichloromethane (DCM) followed by Boc deprotection with the aid of hydrogen chloride in a mixture of dioxane and methanol. The product can be reacted with 4-nitrophenyl chloroformate in the presence of a base such as NMM in an appropriate solvent such as DCM to yield the 4-nitrophenyl carbamate which subsequently can be treated with an amine H-R⁴ in the presence of a base such as DIEA in an appropriate solvent such as THF to give access to the target compound. The final product can be converted to a pharmaceutically acceptable salt as described above.

### Analytical LC-MS

The compounds of the present invention according to formula (I) were analyzed via analytical LC-MS. The conditions used in the analysis are summarized below.

### Analytical conditions summary:

LC10Advp-Pump (Shimadzu) with SPD-M10Avp UV/Vis diode array detector and QP2010
MS-detector in ESI+ modus with UV-detection at 214, 254 and 275 nm,
Column: Waters XTerra MS C18, 3.5 µm, 2.1 * 100 mm,
linear gradient with acetonitrile in water (0.1 % HCOOH)
Flow rate of 0,4 ml/min;

| | |
|---|---|
| Mobile Phase A: | water (0.1 % HCOOH) |
| Mobile Phase B: | acetonitrile (0.1 % HCOOH) |

### Gradient A:

linear gradient from 1% to 95% acetonitrile in water (0.1% HCOOH)

| | |
|---|---|
| 0.00 min | 1%B |
| 10.00 min | 95% B |
| 10.10 min | 99% B |
| 11.40 min | 99% B |
| 11.50 min | 1%B |
| 13.00 min | Pump STOP |

### Gradient B:

linear gradient from 1% to 95% acetonitrile in water (0.1% HCOOH)

| | |
|---|---|
| 0.00 min | 1%B |
| 5.00 min | 95 % B |
| 5.10 min | 99 % B |
| 6.40 min | 99 % B |
| 6.50 min | 1 % B |
| 8.00 min | Pump STOP |

### Gradient C:

linear gradient from 5% to 95% acetonitrile in water (0.1% HCOOH)

| | |
|---|---|
| 0.00 min | 5% B |
| 10.00 min | 95% B |
| 10.10 min | 99% B |
| 11.40 min | 99% B |
| 11.50 min | 1 % B |
| 13.00 min | Pump STOP |

### Gradient D:

linear gradient from 5% to 95% acetonitrile in water (0.1 % HCOOH)

| | |
|---|---|
| 0.00 min | 5% B |
| 5.00 min | 95 % B |
| 5.10 min | 99 % B |
| 6.40 min | 99 % B |
| 6.50 min | 1 % B |
| 8.00 min | Pump STOP |

The following tables describe detailed examples of the invention which can be prepared according to the Reaction schemes 1 to 11. These examples are, however, not construed to limit the scope of the invention in any manner.

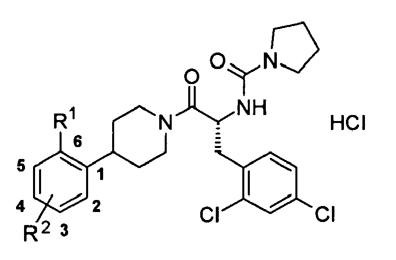

**Table 1:**

| | | | HPLC | | MS | |
|---|---|---|---|---|---|---|
| No. | R¹ | R² | t_{R} (min) | method | MW (calc.) | [M+H]⁺ (found) |
| 1 | 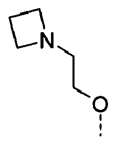 | H | 4.92 | A | 573.57 | 575 |
| 2 | 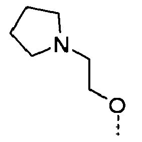 | H | 4.91 | A | 587.60 | 587 |
| 3 | 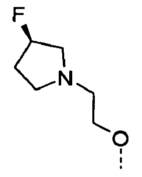 | H | 4.94 | A | 605.59 | 605 |
| 4 | 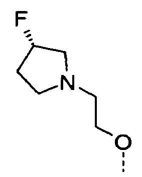 | H | 4.94 | A | 605.59 | 605 |
| 5 | 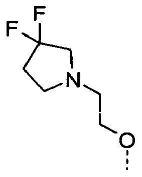 | H | 5.55 | A | 623.58 | 624 |
| 6 | 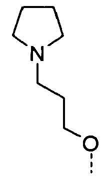 | H | 5.15 | A | 601.63 | 601 |
| 7 | 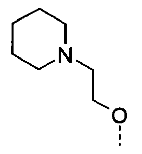 | H | 4.77 | C | 601.63 | 601 |
| 8 | 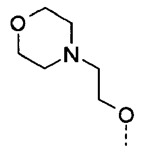 | H | 4.69 | C | 603.60 | 603 |
| 9 | 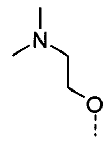 | H | 4.55 | C | 561.56 | 561 |
| 10 | 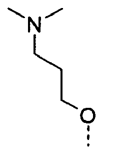 | H | 4.71 | C | 575.59 | 575 |
| 11 | 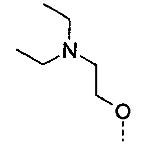 | H | 4.81 | C | 589.61 | 591 |
| 12 | 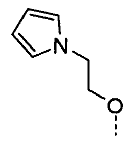 | H | 8.32 | A | 583.57 | 583 |
| 13 | 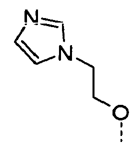 | H | 4.74 | C | 584.55 | 584 |
| 14 | 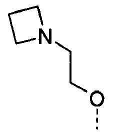 | 4-Cl | 5.04 | C | 608.02 | 609 |
| 15 | 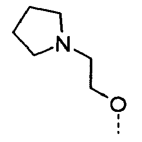 | 3-F | 4.76 | C | 605.59 | 605 |
| 16 | 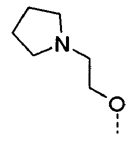 | 3-Cl | 5.18 | C | 622.04 | 621 |
| 17 | 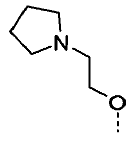 | 4-F | 4.94 | C | 605.59 | 605 |
| 18 | 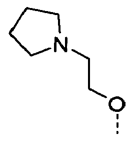 | 4-Cl | 5.23 | C | 622.04 | 621 |
| 19 | 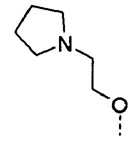 | 3-F 4-F | 5.27 | A | 623.58 | 622 |
| 20 | 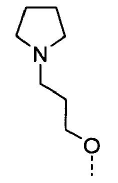 | 4-Cl | 5.31 | C | 636.07 | 635 |
| 21 | 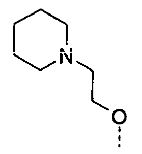 | 4-Cl | 5.40 | A | 636.07 | 635 |
| 22 | 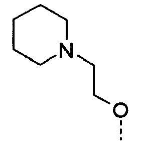 | 3-F 4-F | 5.36 | A | 637.61 | 637 |
| 23 | 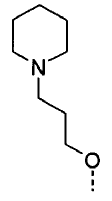 | 4-Cl | 5.49 | C | 650.10 | 649 |
| 24 | 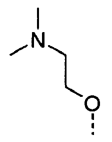 | 4-Cl | 5.23 | A | 596.00 | 595 |
| 25 | 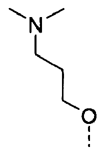 | 4-Cl | 5.24 | C | 610.03 | 609 |
| 26 | 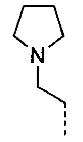 | H | 4.99 | A | 571.60 | 571 |
| 27 | 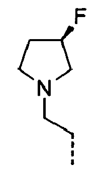 | H | 4.98 | A | 589.59 | 589 |
| 28 | 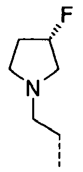 | H | 4.95 | A | 589.59 | 589 |
| 29 | 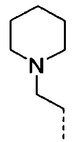 | H | 5.06 | A | 585.63 | 585 |
| 30 | 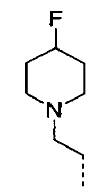 | H | 5.06 | A | 603.62 | 603 |
| 31 | 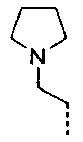 | 3-F | 5.04 | A | 589.59 | 589 |
| 32 | 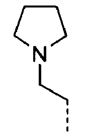 | 4-F | 5.00 | A | 589.59 | 589 |
| 33 | 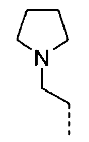 | 4-Cl | 5.26 | A | 606.04 | 605 |
| 34 | 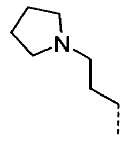 | H | 5.17 | A | 585.63 | 585 |
| 35 | 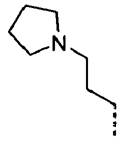 | 4-F | 5.29 | A | 603.62 | 603 |
| 36 | 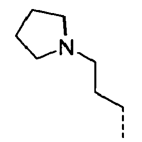 | 4-Cl | 5.39 | A | 620.07 | 619 |

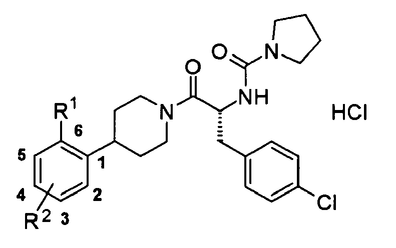

**Table 2:**

| | | | HPLC | | MS | |
|---|---|---|---|---|---|---|
| No. | R¹ | R² | t_{R} (min) | method | MW (calc.) | [M+H]⁺ (found) |
| 37 | 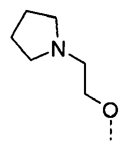 | H | 4.50 | C | 553.15 | 553 |

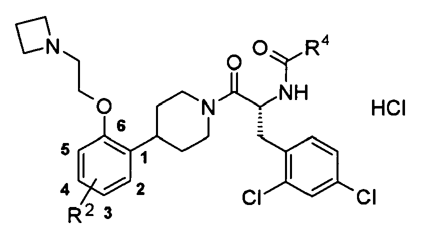

**Table 3:**

| | | | HPLC | | MS | |
|---|---|---|---|---|---|---|
| No. | R² | R⁴ | t_{R} (min) | method | MW (calc.) | [M+H]⁺ (found) |
| 38 | H | 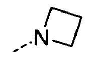 | 4.82 | A | 559.54 | 560 |
| 39 | H | 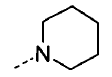 | 5.18 | A | 587.60 | 588 |

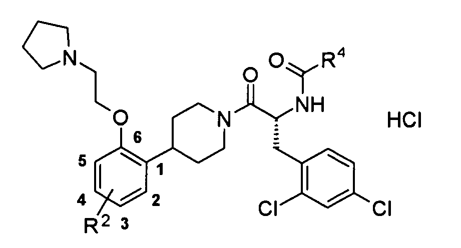

**Table 4:**

| | | | HPLC | | MS | |
|---|---|---|---|---|---|---|
| No. | R² | R⁴ | t_{R} (min) | method | MW (calc.) | [M+H]⁺ (found) |
| 40 | H | 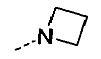 | 4.52 | C | 573.57 | 573 |
| 41 | H | 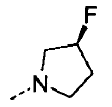 | 4.64 | C | 605.59 | 605 |
| 42 | H | 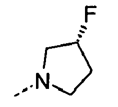 | 4.63 | C | 605.59 | 605 |
| 43 | H | 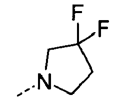 | 4.88 | C | 623.58 | 623 |
| 44 | H | 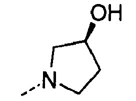 | 4.22 | C | 603.60 | 603 |
| 45 | H | 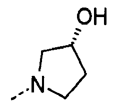 | 4.22 | C | 603.60 | 603 |
| 46 | H | 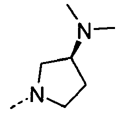 | 3.55 | C | 630.67 | 630 |
| 47 | H | 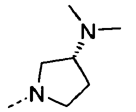 | 3.54 | C | 630.67 | 630 |
| 48 | H | 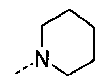 | 4.98 | C | 601.63 | 601 |
| 49 | H | 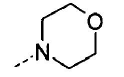 | 4.42 | C | 603.60 | 603 |
| 50 | H | 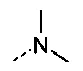 | 4.48 | C | 561.56 | 561 |
| 51 | H | 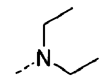 | 4.91 | C | 589.61 | 589 |
| 52 | 4-Cl | 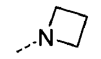 | 5.15 | A | 608.02 | 608 |
| 53 | 4-Cl | 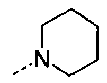 | 5.52 | A | 636.07 | 635 |

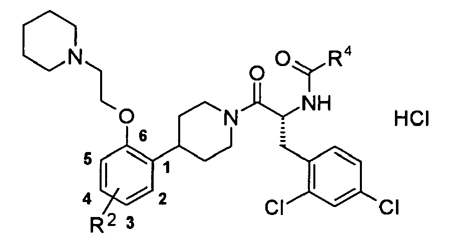

**Table 5:**

| | | | HPLC | | MS | |
|---|---|---|---|---|---|---|
| No. | R² | R⁴ | t_{R} (min) | method | MW (calc.) | [M+H]⁺ (found) |
| 54 | H | 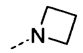 | 4.62 | C | 587.60 | 587 |
| 55 | H | 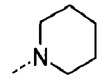 | 5.08 | C | 615.65 | 615 |

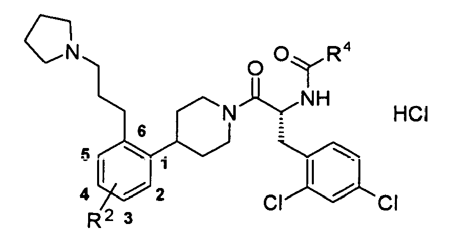

**Table 6:**

| | | | HPLC | | MS | |
|---|---|---|---|---|---|---|
| No. | R² | R⁴ | t_{R} (min) | method | MW (calc.) | [M+H]⁺ (found) |
| 56 | H | 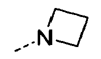 | 4.89 | A | 571.60 | 571 |
| 57 | H | 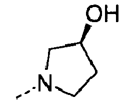 | 4.63 | A | 601.63 | 601 |
| 58 | H | 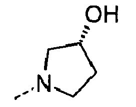 | 4.64 | A | 601.63 | 601 |
| 59 | H | 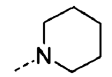 | 5.31 | A | 599.65 | 599 |
| 60 | H | 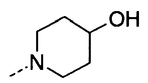 | 4.63 | A | 615.65 | 615 |
| 61 | H | 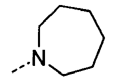 | 5.57 | A | 613.68 | 613 |
| 62 | H | 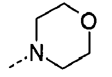 | 4.84 | A | 601.63 | 601 |
| 63 | 4-F | 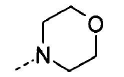 | 5.04 | A | 619.61 | 619 |

The following examples are provided to illustrate the invention and are not limiting the scope of the invention in any manner.

### Synthesis of B-C Moieties:

### B-C Moiety 1:

### Intermediate A1):

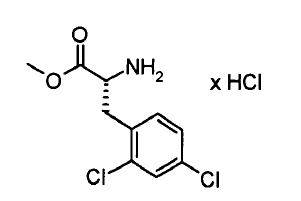

To a suspension of D-2,4-dichlorophenylalanine (10.00 g) in methanol (100 ml) was added dropwise thionylchloride (9.39 ml). During the course of the addition a clear solution was formed and the reaction started to reflux. The reaction mixture was kept under reflux for 2 h. After cooling to room temperature the mixture was evaporated to dryness at 40 °C. The crude product was triturated in diethyl ether, and the insoluble compound was filtered off, washed with diethyl ether, and finally dried in vacuo at room temperature over P₂O₅ overnight. The product was obtained in form of colorless needles.

### Intermediate B1):

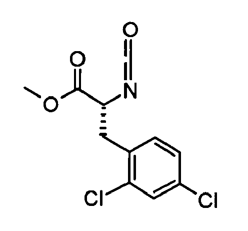

A 350 ml three-necked, flat-bottomed flask was equipped with a mechanical stirrer and charged with DCM (80 ml), saturated aqueous sodium bicarbonate solution (80 ml), and intermediate A1) (5.69 g). The biphasic mixture was cooled in an ice bath and stirred mechanically while triphosgene (1.96 g) was added in a single portion. The reaction mixture was stirred in the ice bath for 45 min and then poured into a 250 ml separatory funnel. The organic layer was collected, and the aqueous layer was extracted with three 20 ml portions of DCM. The combined organic layer was washed with water, dried over Na₂SO₄, filtered, and evaporated in vacuo to dryness to yield the crude product as a semisolid. The residue was purified by Kugelrohr distillation (200-240 °C, 0.04-0.08 mbar). The product was obtained as clear colorless oil.

### Intermediate C1):

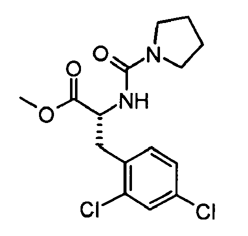

To an ice cooled solution of intermediate B1) (4.99 g) in DCM (50 ml) was added pyrrolidine (4.56 ml). After 10 minutes the ice bath was removed and stirring was continued for 4 h. The reaction mixture was evaporated in vacuo. The residue was redissolved in EtOAc and the organic layer was washed with 1 N HCl, water, sat. Na₂CO₃, water and brine. All the aqueous layers were extracted with EtOAc. The combined organic layer was dried over Na₂SO₄ and evaporated in vacuo to dryness.

### B-C Moiety 1:

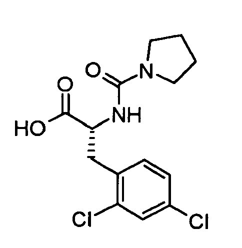

intermediate C1) (6.28 g) was dissolved in MeOH (100 ml) and THF (30 mi) at 0 °C. A solution of lithium hydroxide monohydrate (1.53 g) in water (30 ml) was added dropwise over the course of 5 min. The mixture was stirred at 0 °C for 60 min and then acidified by adding 0.5 M HCl. The reaction mixture was extracted two times with EtOAc. The combined organic layer was washed two times with water and with brine, dried over Na₂SO₄ and evaporated in vacuo. The solid residue was triturated in Et₂O, then filtered off and washed with Et₂O. The product was obtained as a white solid.

### B-C Moiety 2:

### Intermediate A2):

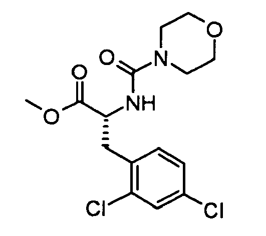

To an ice cooled solution of intermediate B1) (1.00 g) in DCM (10 ml) was added morpholine (954 µl). After 10 minutes the ice bath was removed and stirring was continued for 4 h. The reaction mixture was evaporated in vacuo. The residue was redissolved in EtOAc and the organic layer was washed with 1 N HCl, water, sat. Na₂CO₃, water and brine. All the aqueous layers were extracted with EtOAc. The combined organic layer was dried over Na₂SO₄ and evaporated in vacuo to dryness.

### B-C Moiety 2:

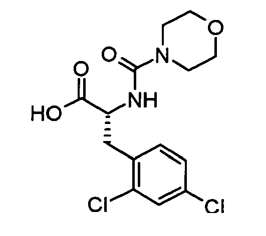

Intermediate A2) (1.26 g) was dissolved in MeOH (20 ml) and THF (6 ml) at 0 °C. A solution of lithium hydroxide monohydrate (293 mg) in water (6 ml) was added dropwise over the course of 5 min. The mixture was stirred at 0 °C for 60 min and then acidified by adding 0.5 M HCl. The reaction mixture was extracted two times with EtOAc. The combined organic layer was washed two times with water and with brine, dried over Na₂SO₄ and evaporated in vacuo. The solid residue was triturated in Et₂O, then filtered off and washed with Et₂O. The product was obtained as a white solid.

All B-C moieties used in this patent application can be prepared using this method starting from an appropriate Boc-protected amino acid and an appropriate amine.
The introduction of basic C moieties was usually achieved using the 4-nitrophenylcarbamate pathway (Reaction scheme 11).

### Synthesis of Example 2:

### Intermediate 2a):

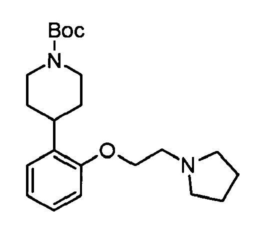

To a solution of 1-Boc-4-(2-hydroxy-phenyl)-piperidine (789 mg) in DMF (15 ml) was added 1-(2-chloroethyl)pyrrolidine hydrochloride (605 mg) and Cs₂CO₃ (3243 mg). The reaction was stirred at room temperature for 18 h. An additional amount of 1-(2-chloroethyl)pyrrolidine hydrochloride (483 mg) and Cs₂CO₃ (926 mg) was added and stirring at room temperature was continued for another 6 h. The reaction mixture was evaporated at 50 °C in vacuo to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated in vacuo to dryness. The residue was finally dried under high vacuum at room temperature overnight.

### Intermediate 2b):

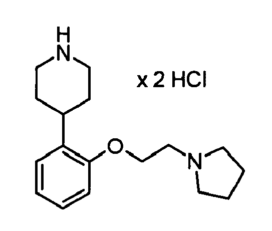

To Boc-protected intermediate 2a) (1002 mg) in methanol (5 ml) was added hydrogen chloride, 4.0 M sol. in 1,4-dioxane (25 ml) and the solution was stirred at room temperature for 2 h. The solvent was removed under reduced pressure. The residue was triturated in acetone (30 ml), filtered off, and washed with acetone (2 x 5 ml). Finally, it was dried in vacuo at room temperature over P₂O₅ overnight to yield a white solid.

### Example 2:

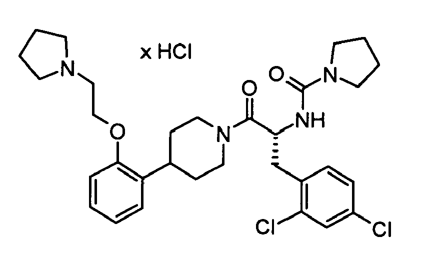

Intermediate 2b) (260 mg), B-C Moiety 1 (310 mg), and HOBt (172 mg) were dissolved in DCM (10 ml). NMM (227 µl) was added and the mixture stirred at room temperature for 30 min. EDCI (252 mg) was added, and the reaction stirred at room temperature for another 60 min. An additional amount of NMM (62 µl) was added and stirring continued at room temperature overnight. The reaction mixture was diluted with EtOAc (100 ml) and washed with sat. Na₂CO₃ (3 x 30 ml), water (2 x 20 ml) and brine (25 ml). The organic layer was dried over Na₂SO₄ and evaporated in vacuo. The crude product was purified by flash chromatography. The purified product was dissolved in EtOAc (3.00 ml), treated with 1 M HCl in Et₂O (633 µl), and the resulting suspension was diluted with hexane (20 ml). The precipitate was filtered off, washed with hexane (5 ml), and dried in vacuo at room temperature over P₂O₅ overnight. The product was obtained as a white solid.

### Synthesis of Example 3:

### Intermediate 3a):

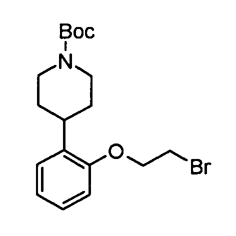

A solution of 1-Boc-4-(2-hydroxy-phenyl)-piperidine (1110 mg), 2-bromoethanol (565 µl), and triphenylphosphine (2100 mg) in THF (40 ml) under argon, was cooled in ice/H₂O. DEAD (ca. 40% in toluene, 3666 µl) was added dropwise, at a rate to keep the temperature below 5 °C (ca. 25 min). After stirring for another 15 min in ice/H₂O, the cooling bath was removed and the mixture was stirred at room temperature overnight. Finally, the mixture was heated in an oil bath (45 °C) for 4 h. The reaction mixture was cooled down to room temperature and then evaporated to dryness in vacuo at 40 °C. The crude product was purified by flash chromatography to yield a slightly yellowish clear oil.

### Intermediate 3b):

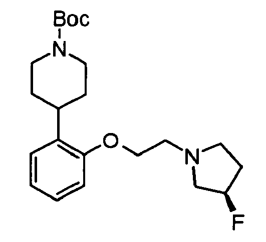

A suspension of intermediate 3a) (130 mg), (R)-2-fluoropyrrolidine hydrochloride (90 mg) and potassium carbonate (234 mg) in MeCN (5 mi) in a tightly capped flask was heated at 45°C in an oil-bath for 48 h. The reaction mixture was diluted with EtOAc (25 ml), filtered, and the filtrate was evaporated in vacuo. The product was purified by flash chromatography.

### Intermediate 3c):

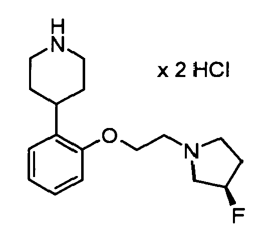

To Boc-protected intermediate 3b) (105 mg) in methanol (1 ml) was added hydrogen chloride, 4.0 M sol. in 1,4-dioxane (5 ml) and the solution was stirred at room temperature for 2 h. The solvent was removed under reduced pressure. The residue was triturated in acetone (6 ml), filtered off, and washed two times with acetone. Finally, it was dried in vacuo at room temperature over P₂O₅ overnight to yield a white solid.

### Example 3:

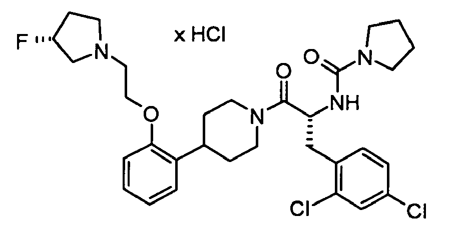

Intermediate 3c) (30 mg), B-C Moiety 1 (36 mg), and HOBt (19 mg) were dissolved in DCM (2.5 ml). NMM (26 µl) was added and the mixture stirred at room temperature for 30 min. EDCI (29 mg) was added, and the reaction stirred at room temperature for another 60 min. An additional amount of NMM (7 µl) was added and stirring continued at room temperature overnight. The reaction mixture was evaporated in vacuo, diluted with EtOAc, washed with sat. Na₂CO₃, water and brine. The aqueous layers were extracted with EtOAc. The combined organic layer was dried over Na₂SO₄, filtered and evaporated in vacuo to dryness. The residue was purified by flash chromatography. The purified product was dissolved in ethyl acetate (300 µl), and treated with 1 M HCl in Et₂O (26 µl) followed by hexane (3 ml). The precipitated salt was filtered off, washed with hexane (1 ml), and finally dried in vacuo at room temperature over P₂O₅ overnight.

### Synthesis of Example 13:

### Intermediate 13a):

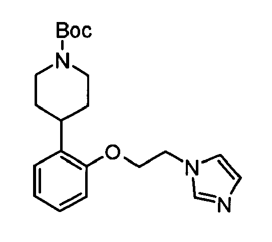

To a solution of 1-Boc-4-(2-hydroxy-phenyl)-piperidine (500 mg) in DMF (7.5 ml) was added 1-(2-chloroethyl) imidazole hydrochloride (680 mg) and Cs₂CO₃ (2060 mg). The reaction was stirred at room temperature for 18 h. An additional amount of 1-(2-chloroethyl) imidazole hydrochloride (300 mg) and Cs₂CO₃ (590 mg) was added and stirring at room temperature was continued for another 74 h. The reaction mixture was evaporated in vacuo to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated in vacuo to dryness. The residue was finally dried under high vacuum at room temperature overnight and purified by flash chromatography.

### Intermediate 13b):

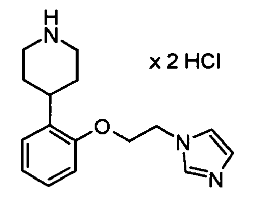

To Boc-protected intermediate 13a) (680 mg) in methanol (5 ml) was added hydrogen chloride, 4.0 M sol. in 1,4-dioxane (10 ml) and the solution was stirred at room temperature for 2 h. The solvent was removed under reduced pressure. The residue was triturated in acetone (30 ml), filtered off, and washed with acetone and diethyl ether. Finally, it was dried in vacuo at room temperature over P₂O₅ overnight to yield a white solid.

### Example 13:

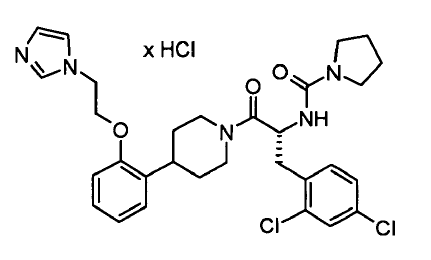

Intermediate 13b) (30 mg), B-C Moiety 1 (26 mg), and HOBt (14 mg) were dissolved in DCM (2 ml). NMM (19 µl) was added and the mixture stirred at room temperature for 30 min. EDCI (21 mg) was added, and the reaction stirred at room temperature for another 60 min. An additional amount of NMM (5 µl) was added and stirring continued at room temperature overnight. The reaction mixture was evaporated in vacuo, diluted with EtOAc, washed with sat. Na₂CO₃, water and brine. The aqueous layers were extracted with EtOAc. The combined organic layer was dried over Na₂SO₄, filtered and evaporated in vacuo to dryness. The residue was purified by flash chromatography. The purified product was dissolved in DCM, and treated with 1 M HCl in Et₂O (27 µl) and evaporated in vacuo to yield the hydrochloride as clear colorless oil.

### Synthesis of Example 20:

### Intermediate 20a):

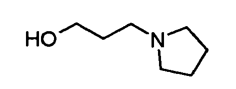

A suspension of pyrrolidine (8.35 ml), 3-bromo-1-propanol (8.67 ml), and potassium carbonate (17.28 g) in MeCN (100 ml) was heated under reflux overnight. The reaction mixture was filtered and evaporated in vacuo. The residue was partitioned between EtOAc (100 ml) and 1 M HCl (50 ml). The organic layer was separated and extracted with 1 M HCl (2 x 25 ml). The combined acidic extract was adjusted to pH 13 with solid KOH, while cooling in ice/H₂O. The resulting clear, slightly yellowish solution was extracted with DCM (5 x 50 ml). The combined organic extract was dried over Na₂SO₄ and evaporated in vacuo. The crude product was purified by vacuum distillation employing a 5 cm *Vigreux* column at a pressure of ca. 15 mbar (with an oil-bath temperature of ca. 120 °C). The fraction distilling off at 89-90 °C was collected. The product was obtained as a colorless oil.

### Intermediate 20b):

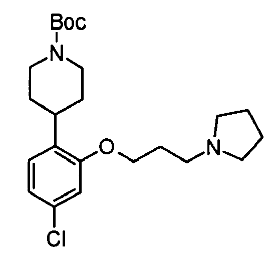

A solution of intermediate 23d) (468 mg), intermediate 20a) (388 mg), and triphenylphosphine (787 mg) in THF (15 ml) under argon, was cooled in ice/H₂O. DEAD (ca. 40% in toluene, 1375 µl) was added dropwise, at a rate to keep the temperature below 5 °C (ca. 15 min). After stirring for another 10 min in ice/H₂O, the cooling bath was removed and the mixture was stirred at room temperature overnight. The reaction mixture was evaporated to dryness in vacuo at 40 °C. The crude product was purified by flash chromatography to yield a clear yellowish oil.

### Intermediate 20c):

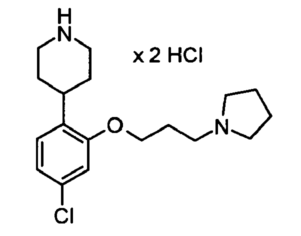

To Boc-protected intermediate 20b) (635 mg) in methanol (3 ml) was added hydrogen chloride, 4.0 M sol. in 1,4-dioxane (11 ml) and the solution was stirred at room temperature for 2 h. The solvent was removed under reduced pressure. The residue was triturated in acetone and diethyl ether, filtered off, and washed with diethyl ether. Finally, it was dried in vacuo at room temperature over P₂O₅ overnight to yield a beige solid.

### Example 20:

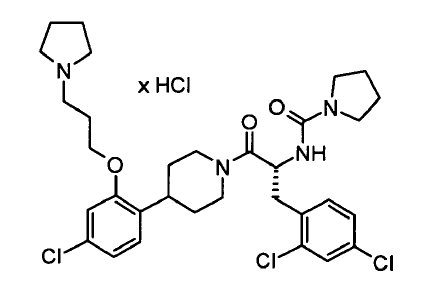

Intermediate 20c) (30 mg), B-C Moiety 1 (31 mg), and HOBt (17 mg) were dissolved in DCM (2.5 ml). NMM (23 µl) was added and the mixture stirred at room temperature for 30 min. EDCI (25 mg) was added, and the reaction stirred at room temperature for another 60 min. An additional amount of NMM (6 µl) was added and stirring continued at room temperature overnight. The reaction mixture was evaporated in vacuo, diluted with EtOAc, washed with sat. Na₂CO₃, water and brine. The aqueous layers were extracted with EtOAc. The combined organic layer was dried over Na₂SO₄, filtered and evaporated in vacuo to dryness. The residue was purified by flash chromatography. The purified product was dissolved in DCM and treated with 1 M HCl in Et₂O (73 µl) and evaporated in vacuo. The residue was dissolved in DCM and the salt was precipitated by addition of Et₂O and hexane. The precipitate was filtered off, washed with hexane and Et₂O and dried in vacuo at 40 °C for 2 hours. The product was obtained as a white solid.

### Synthesis of Example 22:

### Intermediate 22a):

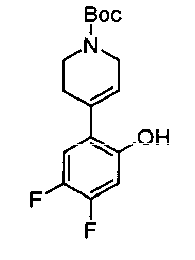

2-Bromo-4,5-difluorophenol (2857 µl), N-Boc-1,2,3,6-tetrahydropyridine-4-boronic acid pinacol ester (7.73 g), potassium carbonate (10.36 g) and dichloro(1,1'-bis(diphenyl-phosphino)-ferrocene)palladium(II) DCM adduct (1.22 g) were dissolved in DMF (150 ml) in a dry apparatus under argon and the mixture was degassed by bubbling with argon for 30 min. The orange suspension was then heated under argon in an oil bath at 85 °C for 1 day to give a dark purple suspension. The reaction mixture was filtered through Celite and evaporated to dryness in vacuo. The crude product was purified by flash chromatography to yield pale green crystals.

### Intermediate 22b):

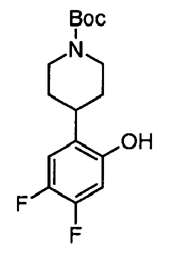

Intermediate 22a) (1404 mg) was dissolved in EtOH (50 ml) and AcOH (50 ml) and platinum(IV) oxide (102 mg) was added. The reaction mixture was evacuated three times and purged with hydrogen. The reaction mixture was then stirred at room temperature for 2 h. The reaction mixture was filtered and evaporated to dryness in vacuo. The residue was coevaporated with toluene (3 x 75 ml) and was finally dried under high vacuum at room temperature overnight to yield a beige solid.

### Intermediate 22c):

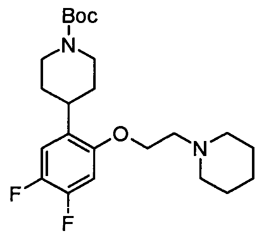

To a solution of intermediate 22b) (674 mg) in DMF (15 ml) was added 1-(2-chloroethyl)piperidine hydrochloride (605 mg) and Cs₂CO₃ (2453 mg). The reaction was stirred at room temperature for 2 days. An additional amount of 1-(2-chloroethyl)piperidine hydrochloride (199 mg) and Cs₂CO₃ (352 mg) was added and stirring at room temperature was continued for another 3 d. The reaction mixture was evaporated at 50 °C in vacuo to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated in vacuo to dryness. The residue was finally dried under high vacuum at room temperature overnight. The crude product was purified by flash chromatography.

### Intermediate 22d):

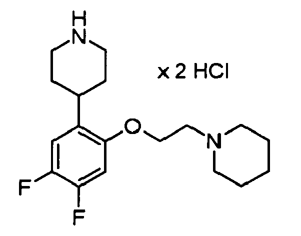

To Boc-protected intermediate 22c) (490 mg) in methanol (2 ml) and dioxane (10 ml) was added hydrogen chloride, 4.0 M sol. in 1,4-dioxane (10 ml) and the solution was stirred at room temperature for 30 min. The solvent was removed under reduced pressure. The residue was triturated in acetone and Et₂O, filtered off, and washed with Et₂O. Finally it was dried in vacuo at room temperature over P₂O₅ overnight to yield an off-white solid.

### Example 22:

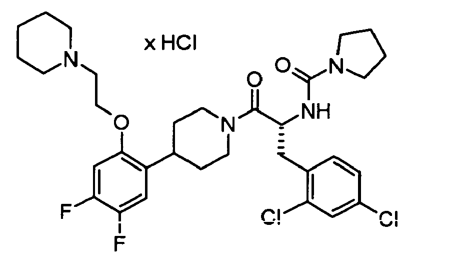

Intermediate 22d) (64 mg), B-C-Moiety 1 (58 mg), and HOBt (27 mg) were dissolved in DCM (2 ml). NMM (26 µl) was added and the mixture stirred at room temperature for 20 min. EDCI (46 mg) was added, and the reaction stirred at room temperature for another 60 min. An additional amount of NMM (20 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated in vacuo. The crude product was purified by flash chromatography. The purified product was dissolved in ethyl acetate (2 ml), treated with 1 M HCl in Et₂O (200 µl), and the resulting suspension was diluted with hexane (20 ml). The precipitate was filtered off, washed with hexane and diethyl ether, and dried in vacuo at room temperature over P₂O₅ overnight. The product was obtained as white solid.

### Synthesis of Example 23:

### Intermediate 23a):

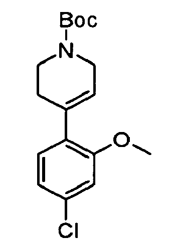

2-Bromo-5-chloroanisole (5.54 g), 1-(2(H)-pyridine-carboxylic acid-3,6-dihydro-4-(4,4,5,5-tetramethyl-1,3,2-dioxaborolan-2-yl)-tert.-butyl ester (7.73 g), dichloro(1,1'-bis(diphenyl-phosphino)-ferrocene)palladium(II) DCM adduct (1.22 g) and K₂CO₃ (10.36 g) were dissolved in degassed DMF in a dry apparatus under argon and the mixture was degassed again by evacuation followed by refilling with argon. The resulting suspension was heated in an oil bath at 85 °C overnight. The mixture was cooled, filtered through Celite and evaporated to dryness. The crude product was purified by flash chromatography to yield a clear yellowish oil.

### Intermediate 23b):

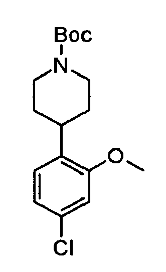

Intermediate 23a) (2.18 g) was dissolved in EtOH (80 ml) and AcOH (80 ml) under argon. Platinum(IV) oxide (0.23 g) was added and the reaction mixture was placed under an H₂ atmosphere using a balloon. The reaction mixture was then stirred at room temperature for 120 min. The reaction mixture was filtered through Celite and evaporated to dryness in vacuo. The residue was coevaporated with toluene (3 x 40 ml). The crude product was purified by flash chromatography to yield a clear colorless oil.

### Intermediate 23c):

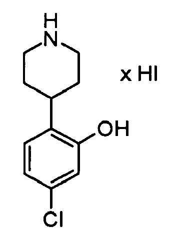

To a solution of intermediate 23b) (1.56 g) in AcOH (6.5 ml) was added hydroiodic acid (5.2 ml of a 57 wt.% aq. solution) and the mixture was heated under reflux (oil bath at 140°C) in an argon atmosphere for 2 h. The reaction mixture was cooled to room temperature and then evaporated to dryness in vacuo. The residue was coevaporated with toluene (3 x 30 ml). The crude product was triturated in Et₂O (40 ml), the insoluble compound was filtered off and washed with Et₂O (10 ml). Finally, the product was dried in vacuo over P₂O₅ at room temperature overnight to yield a white solid.

### Intermediate 23d):

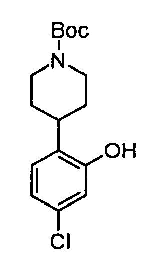

To a solution of intermediate 23c) (1.55 g) in DMF (10 ml) was added DIEA (0.88 ml) followed by di-tert.-butyl-dicarbonate (1.01 g). The reaction mixture was stirred at room temperature for 4 h. The mixture was evaporated in vacuo to dryness and partitioned between 0.5 M HCl (50 ml) and EtOAc (100 ml). The organic layer was washed with water (25 ml) and brine (30 ml). The organic layer was dried with MgSO₄ and evaporated in vacuo to dryness to yield a yellowish solid. The solid residue was triturated in EtOAc (1 ml) and Et₂O (10 ml), and left in the fridge overnight to complete crystallization of the product. The precipitate was then filtered off, washed with cold Et₂O (1 ml), and finally dried in vacuo at room temperature over P₂O₅ overnight. The product was obtained in form of a white solid.

### Intermediate 23e):

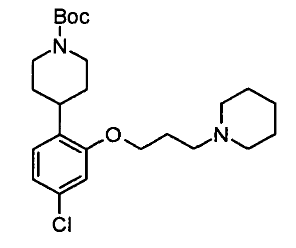

To a solution of intermediate 23d) (468 mg) in DMF (8 mi) was added 1-(3-chloropropyl)piperidine hydrochloride (373 mg) and CS₂CO₃ (1710 mg). The reaction was stirred at room temperature for 18 h. An additional amount of 1-(3-chloropropyl)piperidine hydrochloride (297 mg) and Cs₂CO₃ (489 mg) were added and stirring at room temperature was continued for 3 d. The reaction mixture was evaporated at 40 °C in vacuo to dryness and the residue was partitioned between EtOAc and water. The aqueous layer was extracted with EtOAc. The combined organic layer was washed with water and brine. The organic layer was dried over Na₂SO₄ and evaporated in vacuo to dryness. The crude product was purified using flash chromatography.

### Intermediate 23f):

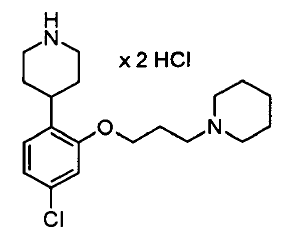

To Boc-protected intermediate 23e) (651 mg) in methanol (3 ml) was added hydrogen chloride, 4.0 M sol. in 1,4-dioxane (11 ml) and the solution was stirred at room temperature for 90 min. The solvent was removed under reduced pressure. The residue was triturated in acetone and Et₂O, filtered off, and washed with Et₂O. Finally, it was dried in vacuo at room temperature over P₂O₅ overnight to yield a white solid.

### Example 23:

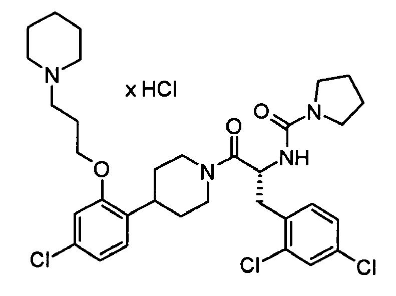

Intermediate 23f) (30 mg), B-C Moiety 1 (30 mg), HOBt (17 mg) were dissolved in DCM (2 ml). NMM (22 µl) was added and the mixture stirred at room temperature for 30 min. EDCI (25 mg) was added, and the reaction stirred at room temperature for another 60 min. An additional amount of NMM (6 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated in vacuo. The crude product was purified by flash chromatography. The purified product was dissolved in DCM, treated with 1 M HCl in Et₂O (66 µl), and the resulting suspension was diluted with diethyl ether and hexane. The precipitate was filtered off, washed with hexane and diethyl ether, and dried in vacuo at room temperature over P₂O₅ overnight. The product was obtained as a white solid.

### Synthesis of Example 30:

### Intermediate 30a):

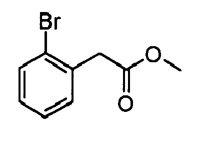

(2-Bromo-phenyl)-acetic acid (5.38 g) was dissolved in methanol (20.26 ml). Then concentrated sulfuric acid (0.27 ml) was added, and the reaction mixture was heated under reflux overnight (oil bath temperature 85 °C) with exclusion of humidity by means of a drying tube (blue silica gel). The reaction mixture was evaporated in vacuo at 40 °C and the colorless oily residue was poured into ice-water (50 ml). The resulting white emulsion was extracted with Et₂O (75 ml), and the organic phase was washed with sat. Na₂CO₃ (3 x 20 ml), H₂O (15 ml), and brine (15 ml). The organic phase was dried with MgSO₄ and evaporated in vacuo to yield a colorless clear oil.

### Intermediate 30b):

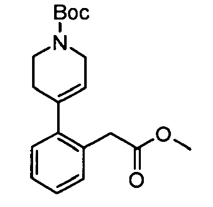

Intermediate 30a) (4.00 g), N-Boc-1,2,3,6-tetrahydropyridine-4-boronic acid pinacol ester (5.40 g), potassium carbonate (7.24 g), and dichloro(1,1'-bis(diphenyl-phosphino)-ferrocene)palladium(II) DCM adduct (860 mg) were dissolved in DMF (150 ml) in a dry apparatus under argon and the mixture was degassed by bubbling with argon for 30 min.

The orange suspension was then heated under argon in an oil bath at 85 °C overnight. The reaction mixture was filtered through Celite and evaporated to dryness in vacuo. The residue was triturated in DCM (50 ml) and the insoluble parts were filtered off. The filtrate was concentrated and subjected to flash chromatography. The product was obtained as clear yellow oil.

### Intermediate 30c):

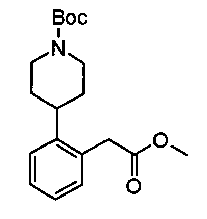

Intermediate 30b) (1.99 g) was dissolved in EtOH (30 ml) and AcOH (30 ml) and platinum(IV) oxide (400 mg) was added. The reaction mixture was evacuated three times and purged with hydrogen. The reaction mixture was then stirred at room temperature for 2 h. The reaction mixture was filtered and evaporated to dryness in vacuo. The residue was coevaporated with toluene (3 x 75 ml) and was finally dried under high vacuum at room temperature overnight.

### Intermediate 30d):

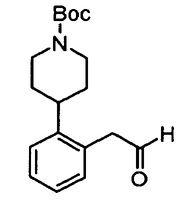

intermediate 30c) (2.85 g) was dissolved in dry diethyl ether (30 ml) under inert atmosphere and cooled to -72°C. At this temperature diiisobutylaluminum hydride, 1.0 M in hexane (12.8 ml) was added dropwise in the course of 30 min. The reaction mixture was stirred at -72°C for 2 h. Methanol (173 µl) was added and the mixture was warmed up to 0°C. Water (1.5 ml) was added and the mixture was filtered through a bed of sodium sulfate. After washing twice with diethyl ether (30 ml each) the combined organic filtrate was concentrated in vacuo. The crude product was purified by flash chromatography to yield a colorless oil.

### Intermediate 30e):

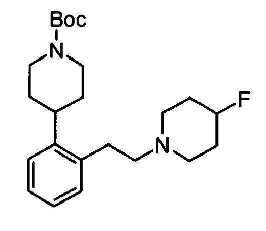

To a solution of the intermediate 30d) (121 mg) and 4-fluoropiperidine hydrochloride (56 mg) in dichloroethane (5 ml), DIEA (139 µl) was added followed by sodium triacetoxyborohydride (119 mg). The reaction mixture was then stirred for 4 h at room temperature. The mixture was diluted with EtOAc (70 ml) and washed two times with sat. NaHCO₃ (25 ml each), water and brine (25 ml each). The organic phase was dried over Na₂SO₄ and concentrated. The crude product was purified using flash chromatography.

### Intermediate 30f):

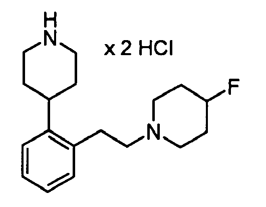

To intermediate 30e) (102 mg) in dioxane (5 ml) and methanol (1 ml) was added hydrogen chloride, 4.0 M sol. in 1,4-dioxane (5 ml) and the solution was stirred for 30 min at room temperature. The solvent was removed under reduced pressure, the residue was triturated with acetone (5 mi) and diethyl ether (25 ml) and the product was filtered off.

### Example 30:

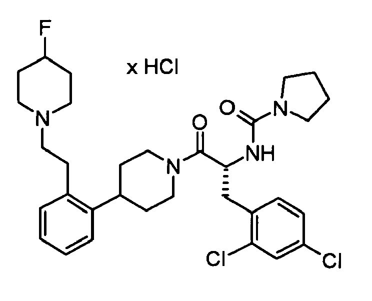

Intermediate 30f) (29 mg), B-C Moiety 1 (28 mg), and HOBt (14 mg) were dissolved in DCM (1 ml). NMM (13 µl) was added and the mixture stirred at room temperature for 20 min. EDCI (23 mg) was added, and the reaction stirred at room temperature for another 60 min. An additional amount of NMM (10 µl) was added and stirring continued at room temperature overnight. The reaction mixture was poured into water (20 ml), diluted with ethyl acetate and the organic phase was separated. The aqueous phase was extracted two times with ethyl acetate. The combined organic phase was washed three times with saturated sodium bicarbonate solution, dried over Na₂SO₄ and concentrated. The residue was purified by flash chromatography. The purified product was dissolved in ethyl acetate and treated with 1 M HCl in Et₂O (100 µl). The product was precipitated by addition of hexane (20 ml). The precipitate was filtered off and dried in vacuo over P₂O₅. The product was obtained as white solid.

### Synthesis of Example 31:

### Intermediate 31a):

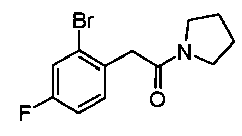

2-Bromo-4-fluorophenylacetic acid (2330 mg), EDCI (2109 mg) and DMAP (100 mg) were dissolved in DCM (100 ml). Pyrrolidine (918 µl) was added and the reaction mixture was stirred overnight. The reaction mixture was poured into water (100 ml) and the organic layer was separated. The aqueous layer was extracted twice with DCM. The combined organics were washed three times with 0.5 N HCl (30 ml each), three times with 1 M sodium hydroxide solution and brine, dried over Na₂SO₄ and the solvent was removed under reduced pressure. The crude product was purified by flash chromatography.

### Intermediate 31b):

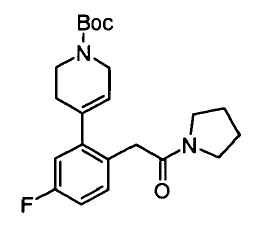

Intermediate 31 a) (1692 mg), N-Boc-1,2,3,6-tetrahydropyridine-4-boronic acid pinacol ester (1920 mg), potassium carbonate (2450 mg), and dichloro(1,1'-bis(diphenyl-phosphino)-ferrocene)palladium(II) DCM adduct (286 mg) were dissolved in DMF (70 ml) in a dry apparatus under argon and the mixture was degassed by bubbling with argon for 30 min. The orange suspension was then heated under Argon in an oil bath at 85 °C overnight. The reaction mixture was filtered through Celite and evaporated to dryness in vacuo. The residue was triturated in DCM (50 ml) and the insoluble parts were filtered off. The filtrate was concentrated and subjected to flash chromatography.

### Intermediate 31c):

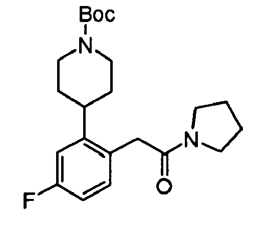

Intermediate 31 b) (2266 mg) was dissolved in EtOH (50 ml) and AcOH (50 ml) and platinum(IV) oxide (132 mg) was added. The reaction mixture was evacuated three times and purged with hydrogen. The reaction mixture was then stirred at room temperature for 2 h. The reaction mixture was filtered and evaporated to dryness in vacuo. The residue was coevaporated with toluene (3 x 75 ml) and was finally dried under high vacuum at room temperature overnight. The crude product was purified by flash chromatography to yield a white solid.

### Intermediate 31d):

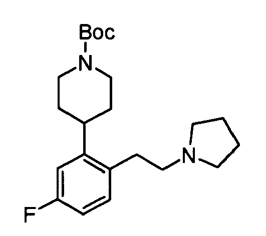

Intermediate 31c) (1392 mg) in diethyl ether (20 ml) was slowly added to a mixture of lithium aluminum hydride (203 mg) and diethyl ether (30 ml) at 0 °C. After addition the reaction mixture was stirred at 0 °C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The product was purified by flash chromatography.

### Intermediate 31e):

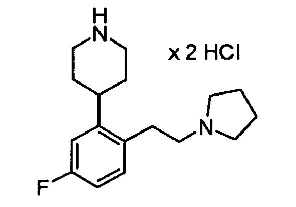

To intermediate 31d) (373 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (10 ml) and diethyl ether (50 ml) and the product was filtered off.

### Example 31:

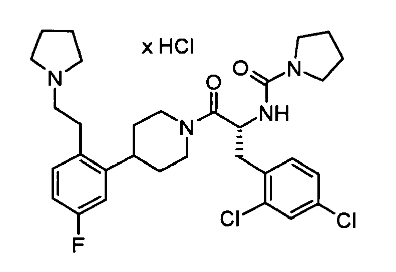

Intermediate 31e) (28 mg), B-C Moiety 1 (28 mg), and HOBt (14 mg) were dissolved in DCM (1 ml). NMM (13 µl) was added and the mixture stirred at room temperature for 20 min. EDCI (23 mg) was added, and the reaction stirred at room temperature for another 60 min. An additional amount of NMM (10 µl) was added and stirring continued at room temperature overnight. The reaction mixture was poured into water (20 ml), diluted with ethyl acetate and the organic phase was separated. The aqueous phase was extracted two times with ethyl acetate. The combined organic phase was washed three times with saturated sodium bicarbonate solution, dried over Na₂SO₄ and concentrated. The residue was purified by flash chromatography. The purified product was dissolved in ethyl acetate and treated with 1M HCl in Et₂O (100 µl). The product was precipitated by addition of hexane (20 ml). The precipitate was filtered off and dried in vacuo over P₂O₅. The product was obtained as a white solid.

### Synthesis of Example 36:

### Intermediate 36a):

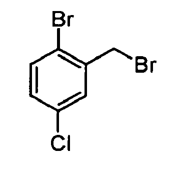

2-Bromo-5-chlorotoluene (8.26 ml) and N-bromosuccinimide (11.03 g) in carbontetrachloride (50 ml) were treated with a catalytic amount of benzoylperoxide (100 mg) and heated under reflux until the reaction had reached completion as monitored by TLC. The reaction mixture was then allowed to cool and filtered. The filtrate was washed twice with water and brine, dried over sodium sulfate and concentrated in vacuo.

### Intermediate 36b):

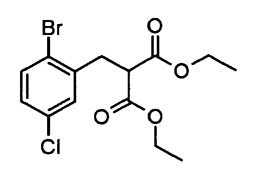

To a solution of sodium ethoxide (2.79 g) in ethanol (30 ml) was added diethyl malonate (6.54 ml) and the mixture was stirred for 1 h at room temperature. The mixture was cooled in an ice-bath and intermediate 36a) (11.67 g) was slowly added and the reaction mixture was kept under reflux overnight. The reaction mixture was evaporated in vacuo and the residue was partioned between diethyl ether and water and the aqueous layer extracted two times with diethyl ether. The combined organic layer was washed twice with water and brine. The combined organic layer was dried over Na₂SO₄ and evaporated in vacuo to dryness. The product was purified by Kugelrohr distillation. The fractions which distilled off between 160 and 230 °C at 0.2-0.3 mbar were collected.

### Intermediate 36c):

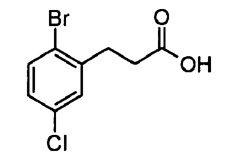

Intermediate 36b) (8.98 g) was heated under reflux in 1.8 M KOH in H₂O/EtO (60 ml) for 5 h. After evaporation of the ethanol an additional amount of KOH (18 g) was added to the residue, and the reaction mixture was stirred for 2 h at 100 °C. The reaction mixture was diluted with 100 ml of H₂O, extracted with Et₂O and the organic layer was washed with H₂O. The combined aqueous layer was cooled with ice/H₂O and acidified with 50 % H₂SO₄ to pH 1. The precipitate was extracted twice with Et₂O (100 ml each) and the organic layers were washed with water and brine. The combined organic layers were dried over Na₂SO₄ and evaporated in vacuo to dryness. The residue was triturated in hexane and less Et₂O, then filtered and washed with hexane and less Et₂O. The solid residue was decarboxylated by heating at 200 °C. The development of CO₂ ceased after 20 min and the melt was cooled to room temperature. The residue was crushed with a glass rod to get a homogeneous beige solid.

### Intermediate 36d):

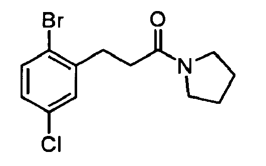

Intermediate 36c) (2320 mg), pyrrolidine (808 µl), EDCI (1856 mg) and DMAP (100 mg) were dissolved in DCM (100 ml) and stirred overnight. The reaction mixture was poured into water (100 ml) and the organic layer was separated. The aqueous layer was extracted twice with DCM. The combined organics were washed three times with with 0.5 N HCl (30 ml each), three times with 1 M sodium hydroxide solution and brine, dried over Na₂SO₄ and the solvent was removed under reduced pressure. The product was obtained as off-white solid after purification by flash chromatography.

### Intermediate 36e):

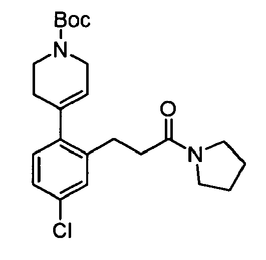

Intermediate 36d) (1901 mg), N-Boc-1,2,3,6-tetrahydropyridine-4-boronic acid pinacol ester (1948 mg), potassium carbonate (3317 mg) and dichloro(1,1'-bis(diphenyl-phosphino)-ferrocene)palladium(II) DCM adduct (294 mg) were dissolved in DMF (70 ml) in a dry apparatus under argon and the mixture was degassed by bubbling with argon for 30 min. The orange suspension was then heated under argon in an oil bath at 85 °C for 3 days to give a dark purple suspension. The reaction mixture was filtered through Celite and evaporated to dryness in vacuo. The crude product was purified by column chromatography.

### Intermediate 36f):

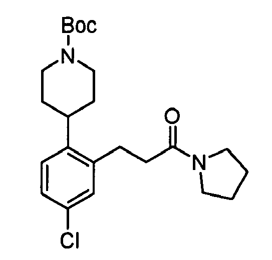

Intermediate 36e) (2372 mg) was dissolved in EtOH (50 ml) and AcOH (50 ml) and platinum(IV) oxide (129 mg) was added. The reaction mixture was evacuated three times and purged with hydrogen. The reaction mixture was then stirred at room temperature for 2 h. The reaction mixture was filtered and evaporated to dryness in vacuo. The residue was coevaporated with toluene (3 x 75 ml) and was finally dried under high vacuum at room temperature overnight.

### Intermediate 36g):

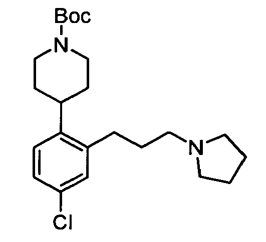

Intermediate 36f) (1687 mg) in diethyl ether (20 ml) was slowly added to a mixture of lithium aluminum hydride (228 mg) and diethyl ether (30 ml) at 0°C. After addition, the reaction mixture was stirred at 0 °C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a colorless oil.

### Intermediate 36h):

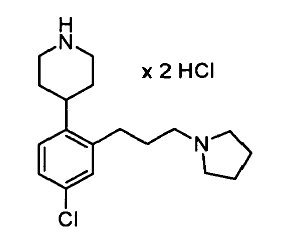

To intermediate 36g) (864 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml) and the product was filtered off. The product was obtained as off-white solid.

### Example 36:

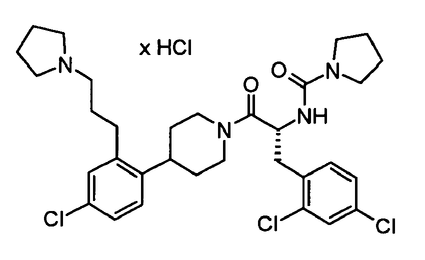

Intermediate 36h) (61 mg), B-C Moiety 1 (58 mg), and HOBt (27 mg) were dissolved in DCM (2 ml). NMM (26 µl) was added and the mixture stirred at room temperature for 20 min. EDCI (46 mg) was added, and the reaction stirred at room temperature for another 60 min. An additional amount of NMM (20 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated in vacuo. The crude product was purified by flash chromatography. The purified product was dissolved in ethyl acetate (2 ml), treated with 1 M HCl in Et₂O (200 µl), and the resulting suspension was diluted with hexane (20 ml). The precipitate was filtered off, washed with hexane and diethyl ether, and dried in vacuo at room temperature over P₂O₅ overnight. The product was obtained as a white solid.

### Synthesis of Example 46:

### Intermediate 46a):

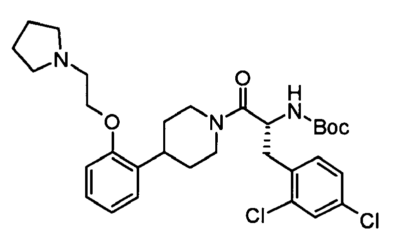

To Boc-D-2,4-dichlorophenylalanine (685 mg) in DCM (20 ml) was added the amine hydrochloride from 2b) (347 mg), N-methylmorpholine (311 µl), HOBt (230 mg) and the mixture was stirred for 30 min. EDCI (351 mg) was added and stirring was continued for 1 h. An additional amount of N-methylmorpholine (91 µl) was added and stirred overnight. The reaction mixture was evaporated in vacuo, diluted with EtOAc, washed with sat. Na₂CO₃, water and brine. The aqueous layers were extracted with EtOAc. The combined organic layers were dried over Na₂SO₄, filtered and evaporated in vacuo to dryness. Purification by flash chromatography yielded the title compound as a white foam.

### Intermediate 46b):

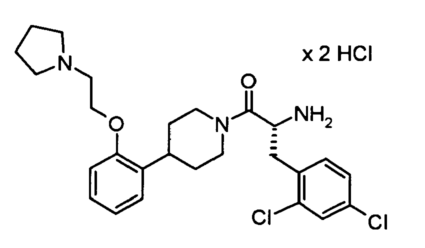

To Boc-protected intermediate 46a) (560 mg) in methanol (5 ml) was added hydrogen chloride, 4.0 M sol. in 1,4-dioxane (9 ml) and the solution was stirred for 90 min at room temperature. The reaction mixture was evaporated in vacuo to dryness. The residue was triturated in Et₂O, filtered off and washed with Et₂O to yield a beige solid.

### Intermediate 46c):

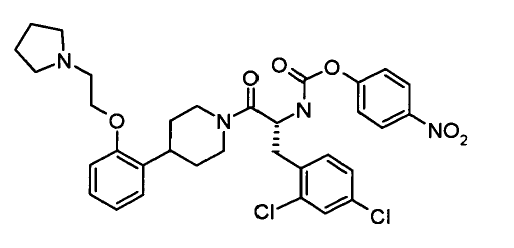

Intermediate 46b) was suspended in DCM (15 ml) and NMM (171 µl) was added. The mixture was cooled in an ice bath with stirring. Then 4-nitrophenyl chloroformate (134 mg) was added, and the reaction mixture was stirred at 0 °C for 60 min. The ice bath was then removed and stirring was continued at room temperature overnight. The reaction mixture was diluted with EtOAc and washed with sat. Na₂CO₃, water, and brine. The aqueous layers were extracted with EtOAc. The combined organic layer was dried over Na₂SOₐ and evaporated in vacuo. The residue was purified by flash chromatography to yield a yellow oil.

### Example 46:

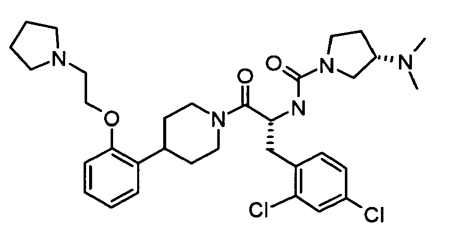

To a solution of intermediate 46c) (25 mg) in THF (1 ml) was added (3S)-3-dimethylaminopyrrolidine (22 mg) and the reaction mixture was stirred at room temperature overnight. The reaction mixture was evaporated in vacuo. The residue was redissolved in EtOAc and the organic layer was washed with sat. Na₂CO₃, water and brine. The aqueous layers were extracted with EtOAc. The combined organic layer was dried over Na₂SO₄ and evaporated in vacuo to dryness. The crude product was purified by flash chromatography. The purified product was dissolved in DCM, treated with 1 M HCl in Et₂O (296 µl), and evaporated in vacuo. The residue was dissolved in DCM and the salt was precipitated by addition of hexane and diethyl ether. The precipitate was filtered off, washed with hexane and dried in vacuo. The product was obtained as a white solid.

### Synthesis of Example 63:

### Intermediate 63a):

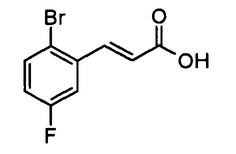

A mixture of 2-bromo-5-fluorobenzaldehyde (10.15 g), malonic acid (5.72 g) and pyridine (1.5 ml) in ethanol (25 ml) was kept under reflux for 7.5 h. After cooling in an ice bath the crystal mass was filtered off. The crystals were washed with cold ethanol (10 ml) and then washed twice with diethyl ether (10 ml each). The residue was suspended in ethanol (60 ml) and kept under reflux for 2-3 h. The mixture was cooled and filtered and the solid was dried under reduced pressure. The product was obtained in form of colorless needles.

### Intermediate 63b):

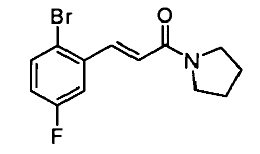

Intermediate 63a) (2451 mg), pyrrolidine (918 µl), EDCI (2109 mg) and DMAP (100 mg) were dissolved in DCM (100 ml) and stirred overnight. The reaction mixture was poured into water (100 ml) and the organic layer was separated. The aqueous layer was extracted twice with DCM. The combined organics were washed three times with with 0.5 N HCl (30 ml each), three times with 1 M sodium hydroxide solution and brine, dried over Na₂SO₄ and the solvent was removed under reduced pressure. The crude product was purified by flash chromatography to yield a white solid.

### Intermediate 63c):

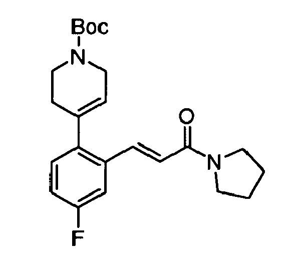

Intermediate 63b) (1130 mg), N-Boc-1,2,3,6-tetrahydropyridine-4-boronic acid pinacol ester (1231 mg), potassium carbonate (1571 mg) and dichloro(1,1'-bis(diphenyl-phosphino)-ferrocene)palladium(II) DCM adduct (188 mg) were dissolved in DMF (50 ml) in a dry apparatus under argon and the mixture was degassed by bubbling with argon for 30 min. The orange suspension was then heated under argon in an oil bath at 85 °C for 1 day. The reaction mixture was filtered through Celite and evaporated to dryness in vacuo. The crude product was purified by flash chromatography to yield a beige solid.

### Intermediate 63d):

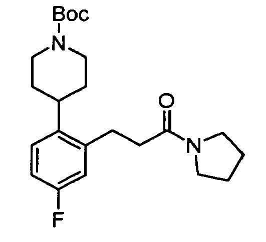

Intermediate 63c) (1459 mg) was dissolved in ethanol (50 ml) and 10% palladium on activated carbon (150 mg) was added. The reaction mixture was purged three times with hydrogen (5 bar) and stirred under hydrogen atmosphere (10 bar) for 3 days. The crude mixture was filtered through Celite and the solvent was removed under reduced pressure to yield a yellow-grey oil.

### Intermediate 63e):

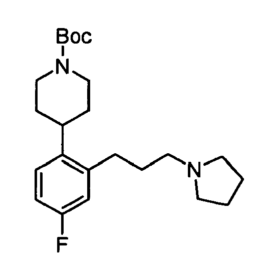

Intermediate 63d) (1472 mg) in diethyl ether (20 ml) was slowly added to a mixture of lithium aluminum hydride (207 mg) and diethyl ether (30 ml) at 0°C. After addition the reaction mixture was stirred at 0 °C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a pale yellow oil.

### Intermediate 63f):

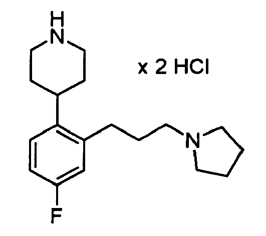

To intermediate 63e) (654 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml) and the product was filtered off to yield an off-white solid.

### Example 63:

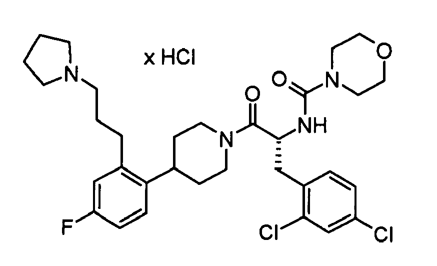

Intermediate 63f) (58 mg), B-C Moiety 2 (61 mg), and HOBt (27 mg) were dissolved in DCM (2 ml). NMM (26 µl) was added and the mixture stirred at room temperature for 20 min. EDCI (46 mg) was added, and the reaction stirred at room temperature for another 60 min. An additional amount of NMM (20 µl) was added and stirring continued at room temperature overnight. The reaction mixture was diluted with EtOAc (50 ml) and washed with sat. Na₂CO₃ (3 x 20 ml), water (2 x 10 ml) and brine (10 ml). The organic layer was dried over Na₂SO₄ and evaporated in vacuo. The crude product was purified with flash chromatography. The purified product was dissolved in ethyl acetate (2 ml), treated with 1 M HCl in Et₂O (200 µl), and the resulting suspension was diluted with hexane (20 ml). The precipitate was filtered off, washed with hexane and diethyl ether, and dried in vacuo at room temperature over P₂O₅ overnight. The product was obtained as a white solid.

### Biological Assays

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4 °C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have IC₅₀ values less than 2 µM.

**Table 7: Biological data for selected examples of the invention**

| Example | hMC-4R binding assay IC₅₀/nM | hMC-4R functional assay EC₅₀/nM | % activation functional assay |
|---|---|---|---|
| SHU-9119 | 1.9 | | 7 |
| NDP-α-MSH | 1.1 | 3.4 | 100 |
| 2 | 3.7 | | 0 |
| 30 | 9.6 | | 0 |
| 43 | 4.4 | | 0 |
| 46 | 3.0 | | 0 |
| 62 | 5.3 | | 0 |

### C. In Vivo Food Intake Models

### 1. Spontaneous Feeding Paradigm

Food intake in rats is measured after i.p. or p.o. administration of the test compound (see e.g. A.S. Chen et al. Transgenic Res 2000 Apr ;9(2):145-154).

### 2. Model of LPS-Induced Anorexia and Tumor-Induced Cachexia

Prevention or amelioration of anorexia induced by either lipopolysaccharide (LPS) administration or cachexia induced by tumor growth is determined upon i.p. or p.o. administration of test compounds to rats (see e.g. D.L. Marks, N. Ling, and R.D. Cone, Cancer Res 2001 Feb 15;61 (4):1432-1438).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in K.E. McKenna et al, A Model For The Study of Sexual Function In Anesthetized Male And Female Rats, Am. J. Physiol. (Regulatory Integrative Comp. Physiol 30): R1276-R1285, 1991; K.E. McKenna et al, Modulation By Peripheral Serotonin of The Threshold For Sexual Reflexes In Female Rats, Pharm. Bioch. Behav., 40:151-156, 1991; and L.K. Takahashi et al, Dual Estradiol Action In The Diencephalon And The Regulation of Sociosexual Behavior In Female Golden Hamsters, Brain Res., 359: 194-207, 1985.

### Examples of a Pharmaceutical Composition

As a specific embodiment of an oral composition of a compound of the present invention, 30 mg of Example 2 is formulated with sufficient finely divided lactose to provide a total amount of 580 to 590 mg to fill a size 0 hard gelatin capsule.

As another specific embodiment of an oral composition of a compound of the present invention, 25 mg of Example 18 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound according to formula (I) 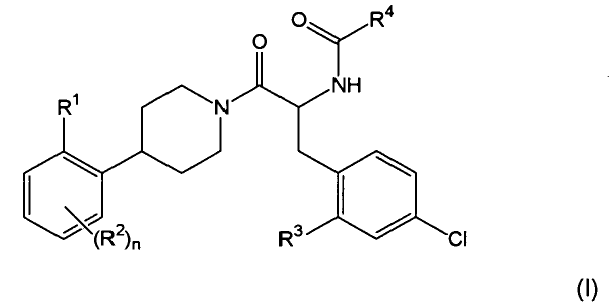 and the enantiomers, diastereomers, tautomers, solvates and pharmaceutically acceptable salts thereof,
wherein
R¹ is -(CH₂)ₗ-T,
-O-(CH₂)ₘ-T
T is NR⁵R⁶,
morpholine, 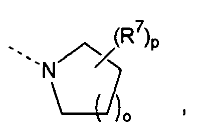 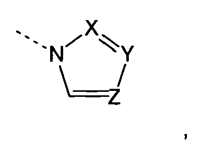
R⁵ and R⁶ are independently
C₁₋₆-alkyl,
C₂₋₆-alkenyl
C₂₋₆-alkinyl,
C₂₋₆-alkylene-O-C₁₋₆-alkyl
R⁷ is halogen,
CN
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH
X is CH, N,
Y is CH, N,
Z is CH, N,
R² is F
Cl,
methyl,
CF₃
R³ is H,
Cl,
methyl,
R⁴ is NR⁸R⁹, 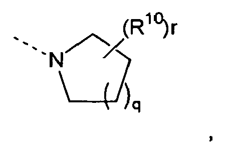 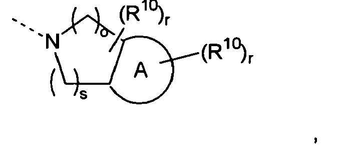
morpholine, optionally substituted by 1 to 3, same or different substituents R¹¹
R⁸ and R⁹ are independently
C₁₋₆-alkyl,
C₂₋₆-alkenyl,
C₂₋₆-alkinyl,
C₂₋₆-alkylene-O-C₁₋₆-alkyl,
R¹⁰ is halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl),
-N(C₁₋₆-alkyl)₂,
R¹¹ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl,
C₁₋₆-alkylene-N(C₁₋₆-alkyl)₂,
A is a 3-7-membered saturated, unsaturated or aromatic ring containing 0-2 nitrogen atoms,
I is 1, 2, 3, 4,
m is 2, 3, 4,
n is 0, 1, 2, 3, 4,
o is 0, 1, 2,
p is 0, 1, 2, 3, 4,
q is 0, 1, 2, 3,
r is 0, 1, 2, 3, 4 and
s is 1,2.

2. The compound of claim 1 according to formula (I') 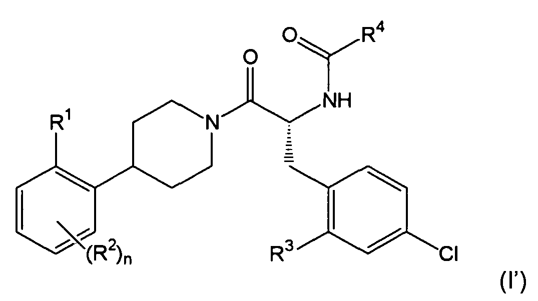 wherein R¹, R², R³, R⁴ and n are as defined in claim 1.

3. The compound of claim 1 or 2, wherein at least one of R⁵ and R⁶ is selected from
C₂₋₆-alkenyl,
C₂₋₆-alkinyl, and
C₂₋₆-alkylene-O-C₁₋₆-alkyl.

4. The compound of any of claims 1 to 3, wherein
R² is F or Cl, and
R³ is Cl.

5. The compound of any of claims 1 to 4, wherein
I is 2 or 3, and
m is 2 or 3.

6. The compound of any of claims 1 to 5 as medicament.

7. Use of the compound of any of claims 1 to 5 for the preparation of a medicament for the treatment or prophylaxis of disorders, diseases or conditions responsive to the inactivation or activation of the melanocortin-4 receptor in a mammal.

8. Use according to claim 7 for the preparation of a medicament for the treatment or prophylaxis of cancer cachexia.

9. Use according to claim 7 for the preparation of a medicament for the treatment or prophylaxis of muscle wasting.

10. Use according to claim 7 for the preparation of a medicament for the treatment or prophylaxis of anorexia.

11. Use according to claim 7 for the preparation of a medicament for the treatment or prophylaxis of anxiety and/or depression.

12. Use according to claim 7 for the preparation of a medicament for the treatment or prophylaxis of obesity.

13. Use according to claim 7 for the preparation of a medicament for the treatment or prophylaxis of diabetes mellitus.

14. Use according to claim 7 for the preparation of a medicament for the treatment or prophylaxis of male or female sexual dysfunction.

15. Use according to claim 7 for the preparation of a medicament for the treatment or prophylaxis of erectile dysfunction.

16. A pharmaceutical composition comprising a compound of any of claims 1 to 5 and a pharmaceutically acceptable carrier.
